# EUROPEAN PATENT SPECIFICATION

(11) **EP 1 562 575 B1**
(45) Date of publication and mention of the grant of the patent: **20.05.2009**
(21) Application number: 03777773.7
(22) Date of filing: 22.10.2003
(51) Int. Cl.: A61K 31/19, A61P 25/08, C07C 61/135, C07C 69/753, C07C 233/58, C07C 237/24, C07C 61/13

(54) **FUSED CYCLOALKYL AMIDES AND ACIDS AND THEIR THERAPEUTIC APPLICATIONS FOR THE TREATMENT OF I.A. EPILEPSY**
FUSIONIERTE CYCLOALKYL AMID- UND CARBONSÄURE-DERIVATE UND DEREN THERAPEUTISCHE VERWENDUNG ZUR BEHANDLUNG VON U.A. EPILEPSIE
CYCLOALKYLAMIDES ET ACIDES CONDENSES ET LEURS APPLICATIONS THERAPEUTIQUES NOTAMMENT DANS LE TRAITEMENT DE L'EPILEPSIE

(30) Priority: 22.10.2002 US 277308
(43) Date of publication of application: 17.08.2005
(73) Proprietor: ABBOTT LABORATORIES, Abbott Park IL 60064-3500 (US)
(72) Inventor: BENNANI, Youssef, L., Shaker Heights, OH 44122 (US); CHANG, Sou-Jen, Prairie View, IL 60069 (US); CHEMBURKAR, Sanjay, R., Gurnee, IL 60031 (US); DART, Michael, J., Highland Park, IL 60035 (US); FERNANADO, Dilinie, P., Gurnee, IL 60031 (US); GRIEME, Timothy, A., Chicago, IL 60640 (US)
(74) Representative: Modiano, Micaela Nadia
(86) International application number: PCT/US2003/033426
(87) International publication number: WO 2004/037241

(56) References cited:
- US-A- 3 725 423
- US-A- 6 124 361
- DATABASE CHEMCATS [Online] Chemical abstracts service, Columbus, Ohio, U.S.; XP002271979 & "ChemDiv, Inc. Product Library" 25 April 2003 (2003-04-25) , CHEMDIV, INC. , SAN DIEGO, U.S.A.
- DATABASE CHEMCATS [Online] Chemical abstracts service, Columbus, Ohio, U.S.; XP002271980 & "Interchim Intermediates Catalog" 9 July 2002 (2002-07-09) , INTERCHIM , MONTLUCON, FRANCE
- DATABASE CROSSFIRE BEILSTEIN [Online] Beilstein Institut zur Förderung der Chemischen Wissenschaften, Frankfurt am Main, DE; Database accession no. BRN 3131956 XP002271981 & MOUSSERON ET AL.: C.R. HEBD. SEANCES ACAD. SCI., vol. 243, 1956, page 1880
- RYNBRANDT, R.H., DUTTON, F.E., SCHMIDT, F.L.: "exo-Bicyclo[3.1.0]hexane-6-carboxylic acid and related compounds, oral hypoglycemic agents" J. MED. CHEM., vol. 15, no. 4, 1972, pages 424-426, XP002271978
- BRANA, M.F. AND MARTINEZ, M.: "Sintesis y actividad farmacologica de homologos ciclicos del acid dipropilacetico (I) [synthesis and pharmacological activity of cyclic homologs of dipropylacetic acid]" ANALES DE QUIMICA, vol. 79, no. 1, 1983, pages 47-51, XP009026593

## Description

### TECHNICAL FIELD

The present invention relates to fused cycloalkyl amides and acids, to the use of these compounds in the manufacture of medicaments to treat epilepsy, bipolar disorder, psychiatric disorders, migraine, pain, movement disorders, and to the use of these compounds in the manufacture of medicaments to provide neuroprotection, and to the preparation of these compounds.

### BACKGROUND OF THE INVENTION

Epilepsy is a common neurological disorder characterized by spontaneous recurrent seizures resulting from abnormal electrical discharges in the brain. It is a health problem that affects roughly 1% of the worldwide population (Loscher, W., Current status and future directions in the pharmacotherapy of epilepsy, Trends Pharmacol. Sci., 2002, 23 (3), 113-118). Epileptic seizures are divided into two major groups, partial or generalized. Partial (focal or local) seizures originate from one or more localized parts of the brain, whereas generalized seizures simultaneously emanate from both brain hemispheres. More than 40 distinct epilepsies have been identified and are characterized by a variety of factors including type of seizure, etiology, age of onset, severity, and EEG features (Commision on Classification and Terminology of the International League Against Epilepsy, Proposal for revised classification of epilepsies and epileptic syndromes, Epilepsia, 1989, 30 (4), 389-399). Epileptic disorders encompass a broad range of severities, extending from a mild and benign condition that readily responds to antiepileptic or anticonvulsant drug (AED) treatment, to a severe, debilitating and even life-threatening condition in which the recurrent seizures remain intractable to drug treatment.

Numerous drugs are now available for the symptomatic treatment of epilepsy. Among these are "first generation" AEDs such as phenytoin, carbamazepine, phenobarbital, and valproate. Several new AEDs or "second generation" drugs such as lamotrigine, topiramate, vigabatrin, felbamate, oxcarbazepine, tiagabine, gabapentin, zonisamide, and levetiracetam have entered the marketplace in the last 15 years (Perucca, E., Clinical pharmacology and therapeutic use of the new antiepileptic drugs, Fundamental & Clinical Pharmacology, 2001, 15,405-417). Although the newer AEDs provide benefits, significant efficacy and safety issues remain (Schmidt, D., The clinical impact of new antiepileptic drugs after a decade of use in epilepsy, Epilepsy Res., 2002, 50(1-2), 21-32; Asconape, J. J., Some common issues in the use of antiepileptic drugs, Seminars in Neurology, 2002, 22(1), 27-39; and Wallace, S. J., Newer antiepileptic drugs: advantages and disadvantages, Brain & Development, 2001, 23, 277-283). For example, roughly one third of epileptic patients continue to have seizures. (Loscher, W. and Schmidt, D., New horizons in the development of antiepileptic drugs, Epilepsy Res., 2002 50 (1-2), 3-16). Thus, an urgent and unmet need exists for new AEDs with improved safety and efficacy.

The mechanisms of action of many AEDs are not well characterized, and for some, completely unknown. However, AEDs manage to strike a balance between inhibitory and excitatory mechanisms within the CNS, which ultimately can prevent seizures. At the cellular level, this antiseizure effect appears to be produced by several mechanisms that are generally divided into three main categories: modulation of voltage-gated ion channels (sodium, calcium, and potassium), indirect or direct enhancement of y-aminobutyric acid (GABA)-mediated inhibitory neurotransmission, and inhibition of excitatory (particularly glutamate-mediated) neurotransmission (Kwan, P., Sills, G., Brodie, M. J., The mechanisms of action of commonly used antiepileptic drugs, Pharmacology & Therapeutics, 2001, 90, 21-34; Soderpalm, B., Anticonvulsants: aspects of their mechanisms of action, Eur. J. Pain, 2002, 6(Suppl A), 3-9). Many AEDs exert their actions through multiple mechanisms. In addition, numerous other less well-characterized mechanisms might also be operative and contribute to the biological activity of these drugs.

Several drugs developed initially as AEDs exhibit beneficial effects in a number of common neurological and psychiatric disorders including bipolar disorders, migraine, neuropathic pain, and movement disorders (Beghi, E., The use of anticonvulsants in neurological conditions other than epilepsy, CNS Drugs, 1999, 11 (1), 61-82). The spectrum of uses for AEDs in psychiatric disorders continues to expand. It has been reported that one third of patients currently taking AEDs do so for the treatment of diverse CNS disorders other than epilepsy (Lopes da Silva, F., Post, R. M., Evaluation and prediction of effects of antiepileptic drugs in a variety of other CNS disorders, Epilepsy Research, 2002, 50(1-2), 191-193). Given the increasingly diverse range of clinical utility being recognized with AEDs, it is likely that new chemical entities, which display broad-spectrum anticonvulsant activity, may also show beneficial effects for the treatment of a variety of neurological and psychiatric disorders.

Several AEDs are used clinically to treat the various aspects of bipolar disorder, which is a chronic, cyclic disease characterized by disruptive mood swings from mania to depression. It is a chronic disorder that affects more than 1% of the US population. Carbamazepine was the first AED utilized to treat bipolar disorder (Brambilla, P., Barale, F., Soares, J. C., Perspectives on the use of anticonvulsants in the treatment of bipolar disorder, International Journal of Neuropsychopharmacology, 2001, 4, 421-446). Valproate has more recently emerged and now competes with lithium as a first-line treatment for patients with bipolar disorder, in particular the manic episodes associated with this illness (Angel, I. and Horovitz, T., Bipolar disorder and valproic acid, Current Opinion in Central & Peripheral Nervous System Investigational Drugs (1999), 1(4), 466-469; Bowden, C. L., Brugger, A. M., Swann, A. C., Calabrese, J. R., Janicak, P. G., Petty, F., Dilsaver, S. C., Davis, J. M., Rush, A. J., Small, J. G., GarzaTrevino, E. S., Risch S. C., Goodnick, P. J., Morris, D. D., Efficacy of divalproex vs lithium and placebo in the treatment of mania. The Depakote Mania Study Group, JAMA, 1994, 271(12), 918-24). Lamotrigine has shown beneficial effects in the treatment of bipolar depression (Muzina, D. J., E1-Sayegh, S., Calabrese, J. R., Antiepileptic drugs in psychiatry-focus on randomized controlled trial, Epilepsy Research, 2002, 50 (1-2), 195-202; Calabrese, J. R., Shelton, M. D., Rapport, D. J., Kimmel, S. E., Bipolar disorders and the effectiveness of novel anticonvulsants, J. Clin. Psychiatry, 2002, 63 (suppl 3), 5-9).

In addition to bipolar disorder, a number of neuropsychiatric syndromes and disorders may be treated with AEDs (Bialer, M., Johannessen, S. I., Kupferberg, H. J., Levy, R. H., Loiseau, P., Perucca, E., Progress report on new antiepileptic drugs: a summary of the sixth eilat conference (EILAT VI), Epilepsy Res. 2002,51,31-71; Fountain, N. B., Dreifuss, F. E., The future of valproate. In: Valproate., Loscher W., Editor. 1999, Birkhauser Verlag, Boston). Such psychiatric disorders include: anxiety and panic disorders, post-traumatic stress disorder, schizophrenia, episodic dyscontrol, substance-abuse-related disorders, impulse control disorders, general agitation associated with a variety of psychiatric disorders and dementias, and behavioral disorders associated with autism.

Migraine is defined as a periodically occurring vascular headache characterized by pain in the head (usually unilateral), nausea and vomiting, photophobia, phonophobia, vertigo and general weakness. It is associated with episodic as well as long-term disability and suffering. Migraine is the most common type of vascular headache and affects as much as 15% of the world's population (Krymchantowski, A. V., Bigal, M. E., Moreira, P. E., New and emerging prophylactic agents for migraine, CNS Drugs, 2002, 16 (9), 611-634). Several AEDs have been shown to be effective in the prevention of migraine including valproate, lamotrigine, gabapentin, and topiramate (Wheeler, S. D., Antiepileptic drug therapy in migraine headache, Current Treatment Options in Neurology, 2002, 4, 383-394; Krymchantowski, A. V., Bigal, M. E., Moreira, P. E., New and emerging prophylactic agents for migraine, CNS Drugs, 2002, 16 (9), 611-634). Many AEDs act by attenuating cellular hyperexcitability and providing a balance between GABAergic inhibition and excitatory amino acid-mediated neuronal excitation, factors that may play a role in the pathophysiology of migraines.

Pain is a common symptom of disease and a frequent complaint with which patients present to physicians. Pain is commonly segmented by duration (acute vs. chronic), intensity (mild, moderate, and severe), and type of pain (nociceptive vs. neuropthic). Neuropathic pain encompasses a wide range of pain syndromes of diverse etiologies and is characterized by a neuronal hyperexcitablility in damaged areas of the nervous system. Diabetic neuropathy, cancer neuropathy, and HIV pain are the most commonly diagnosed types of neuropathic pain. Neuropathic pain also afflicts a significant number of patients suffering from a wide range of other disorders such as trigeminal neuralgia, post-herpetic neuralgia, traumatic neuralgia, phantom limb, as well as numerous other painful disorders of ill-defined or unknown origin. Patients generally respond poorly to traditional pain therapeutic approaches and new drugs with improved efficacy, tolerability, and safety are needed.

Carbamazepine was the first AED examined in controlled trials for neuropathic pain and the results support its use in the treatment of paroxysmal attacks in trigeminal neuralgia, post-herpetic neuralgia, and diabetic neuropathy (Jensen, T. S., Anticonvulsants in neuropathic pain: rationale and clinical evidence, Eur. J. Pain, 2002, 6 (suppl A), 61-68). Among the AEDs examined in controlled trials, gabapentin has clearly demonstrated analgesic effects in treating postherpetic neuralgia and painful diabetic neuropathy (Tremont-Lukats, I. W., Megeff, C., Backonja, M.-M., Anticonvulsants for neuropathic pain syndromes: mechanisms of action and place in therapy, Drugs, 60 (5), 1029-1052). Lamotrigine has demonstrated efficacy in relieving pain in patients with trigeminal neuralgia refractory to other treatments (Backonja, M.-M., Anticonvulsants (antineuropathics) for neuropathic pain syndromes, Clin. J. Pain, 2000, 16, S67-S72). Pregabalin, a follow-on compound to gabapentin, has shown efficacy in clinical trials for diabetic neuropthy. In addition, numerous AEDs display antinociceptive, antiallodynic, or antihyperalgesic activity in animal models relevant to a variety of pain states. Therefor, the potential exists for new AEDs to benefit patients suffering from pain.

AEDs have also been used clinically to treat a variety of movement disorders (Magnus, L., Nonepileptic uses of gabapentin, Epilepsia, 1999, 40 (suppl 6), S66-S72; Fountain, N. B., Dreifuss, F. E., The future of valproate. In: Valproate., Löscher W., Editor. 1999, Birkhauser Verlag, Boston; Cutter, N., Scott, D. D., Johnson, J. C., Whiteneck, G., Gabapentin effect on spacticity in multiple sclerosis, a placebo-controlled, randomized trial, 2000, 81, 164-169), and shown positive effects in animal models of movement disorders (Löscher W., Richter, A., Piracetam and levetiracetam, two pyrrolidone derivatives, exert antidystonic activity in a hamster model of paroxysmal dystonia, Eur. J. Pharmacol., 2000, 391, 251254). Movement disorders include restless leg syndrome, essential tremor, acquired nystagmus, post-anoxic myoclonus, spinal myoclonus, spasticity, chorea, and dystonia.

Many AEDs have demonstrated some evidence of neuroprotective activity in a variety of ischemia models (Pitkanen, A., Efficacy of current antiepileptics to prevent neurodegeneration in epilepsy models, Epilepsy Research, 2002, 50, 141-160). These neuroprotective effects indicate that AEDs could be useful in the treatment of stroke, in mitigating brain damage after recovery from cardiac arrest, and in preventing epileptogenesis.

The present invention relates to compounds that are anticonvulsants and therefore can be used to treat a variety of indications including, but not limited to, epilepsy, bipolar disorder, psychiatric disorders, migraine, pain, movement disorders, and to provide neuroprotection.

### SUMMARY OF THE INVENTION

In its principle embodiment, the present invention relates to a use the manufacture of a medicament for treating migraine, epilepsy, or bipolar disorder in a mammal, particularly in a human, of a therapeutically effective amount of a compound of formula (I) 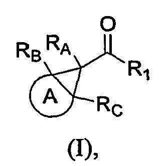 wherein
A is cycloalkyl or bicycloalkyl;
R_{A}, R_{B}, and R_{C} are independently hydrogen or alkyl;
R₁ is OR₂ or NR₃R₄;
R₂ is hydrogen or alkyl;
R₃ and R₄ are independently hydrogen, alkenyl, alkyl, alkynyl, alkoxycarbonylalkyl, aryl, arylalkyl, carboxyalkyl, cycloalkyl, cycloalkylalkyl, heterocycle, heterocyclealkyl, hydroxyalkyl, (NR₅R₆)alkyl, (NR₅R₆)carbonylalkyl, or 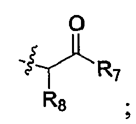 or
R₃ and R₄ taken together with the nitrogen atom to which they are attached form a heterocycle wherein the heterocycle is azepanyl, azetidinyl, aziridinyl, morpholinyl, piperazinyl, piperidinyl, pyrrolidinyl, or thiomorpholinyl;
R₅ and R₆ are independently hydrogen, alkenyl, alkyl, alkynyl, alkoxycarbonylalkyl, aryl, arylalkyl, cycloalkyl, cycloalkylalkyl, heterocycle, heterocyclealkyl, or hydroxyalkyl;
R₇ is alkoxy, alkyl, hydroxy, or -NR₅R₆;
R₈ is alkenyl, alkoxyalkyl, alkoxycarbonylalkyl, alkylthioalkyl, alkynyl, aryl, arylalkyl, carboxyalkyl, cycloalkyl, cycloalkylalkyl, heterocycle, heterocyclealkyl, hydroxyalkyl, mercaptoalkyl, (NR₅R₆)alkyl, (NR₅R₆)carbonylalkyl, or -(CH₂)ₙNHC(=NH)NH₂; and
n is an integer from 1 to 6;
provided that the compound of formula (I) is other than bicyclo[4.1.0]heptane-7-carboxylic acid.

### DETAILED DESCRIPTION OF THE INVENTION

In its principle embodiment, the present invention relates to a use for the manufacture of a medicament for treating migraine, epilepsy, or bipolar disorder in a mammal, particularly in a human, of a therapeutically effective amount of a compound of formula (I) 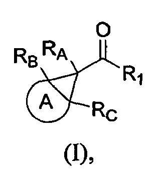 wherein
A is cycloalkyl or bicycloalkyl;
R_{A}, R_{B}, and R_{C} are independently hydrogen or alkyl;
R₁ is OR₂ or NR₃R₄;
R₂ is hydrogen or alkyl;
R₃ and R₄ are independently hydrogen, alkenyl, alkyl, alkynyl, alkoxycarbonylalkyl, aryl, arylalkyl, carboxyalkyl, cycloalkyl, cycloalkylalkyl, heterocycle, heterocyclealkyl, hydroxyalkyl, (NR₅R₆)alkyl, (NR₅R₆)carbonylalkyl, or 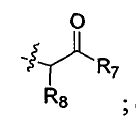 or
R₃ and R₄ taken together with the nitrogen atom to which they are attached form a heterocycle wherein the heterocycle is azepanyl, azetidinyl, aziridinyl, morpholinyl, piperazinyl, piperidinyl, pyrrolidinyl, or thiomorpholinyl;
R₅ and R₆ are independently hydrogen, alkenyl, alkyl, alkynyl, alkoxycarbonylalkyl, aryl, arylalkyl, cycloalkyl, cycloalkylalkyl, heterocycle, heterocyclealkyl, or hydroxyalkyl;
R₇ is alkoxy, alkyl, hydroxy, or NR₅R₆;
R₈ is alkenyl, alkoxyalkyl, alkoxycarbonylalkyl, alkylthioalkyl, alkynyl, aryl, arylalkyl, carboxyalkyl, cycloalkyl, cycloalkylalkyl, heterocycle, heterocyclealkyl, hydroxyalkyl, mercaptoalkyl, (NR₅R₆)alkyl, (NR₅R₆)carbonylalkyl, or -(CH₂)ₙNHC(=NH)NH₂; and
n is an integer from 1 to 6;
provided that the compound of formula (I) is other than bicyclo[4.1.0]heptane-7-carboxylic acid.

In another embodiment, the present invention relates to a treating epilepsy, migraine, or bipolar disorder in a mammal of a therapeutically effective amount of a compound of formula (I) wherein A is cycloalkyl; R₁ is OR₂; and R₂, R_{A}, R_{B}, and R_{C} are as defined in formula (I).

In another embodiment, the present invention relates to a use for the manufacture of a medicament for treating epilepsy, migraine, or bipolar disorder in a mammal of a therapeutically effective amount of a compound of formula (I) wherein A is cycloalkyl wherein the cycloalkyl is optionally substituted with 1 or 2 alkyl groups; R₁ is OR₂; and R₂, R_{A}, R_{B}, and R_{C} are as defined in formula (I).

In another embodiment, the present invention relates to a use for the manufacture of a medicament for treating epilepsy, migraine, or bipolar disorder in a mammal of a therapeutically effective amount of a compound of formula (I) wherein A is cycloalkyl wherein the cycloalkyl is cyclohexane, cycloheptane, cyclooctane, cyclopentane, bicyclo[3.1.1]heptane, or bicyclo[2.2.1]heptane, wherein the cycloalkyl is optionally substituted with 1 or 2 alkyl groups; R₁ is OR₂; R₂ is hydrogen; R_{A} is hydrogen; R_{B} and R_{C} are independently hydrogen or an alkyl group, wherein the preferred alkyl group is methyl.

In another embodiment, the present invention relates to a use for the manufacture of a medicament for treating epilepsy, migraine, or bipolar disorder in a mammal of a therapeutically effective amount of a compound of formula (I) wherein A is bicycloalkyl; R₁ is OR₂; and R₂, R_{A}, R_{B}, and R_{C} are as defined in formula (I).

In another embodiment, the present invention relates to a use for the manufacture of a medicament for treating epilepsy, migraine, or bipolar disorder in a mammal of a therapeutically effective amount of a compound of formula (I) wherein A is bicycloalkyl wherein the bicycloalkyl is optionally substituted with 1 or 2 alkyl groups; R₁ is OR₂; and R₂, R_{A}, R_{B}, and R_{C} are as defined in formula (I).

In another embodiment, the present invention relates to a use for the manufacture of a medicament for treating epilepsy, migraine, or bipolar disorder in a mammal of a therapeutically effective amount of a compound of formula (I) wherein A is bicycloalkyl wherein the bicycloalkyl is bicyclo[4.1.0]heptane, octahydro-1H-4,7-methanoindene, bicyclo[3.2.0]heptane, or octahydropentalene, wherein the bicycloalkyl is optionally substituted with 1 or 2 alkyl groups; R₁ is OR₂; R₂ is hydrogen; R_{A} is hydrogen; R_{B} and R_{C} are independently hydrogen or an alkyl group, wherein the preferred alkyl group is methyl.

In another embodiment, the present invention relates to a use for the manufacture of a medicament for treating epilepsy, migraine, or bipolar disorder in a mammal of a therapeutically effective amount of a compound of formula (I) wherein A is cycloalkyl; R₁ is NR₃R₄; and R₃, R₄, R_{A}, R_{B}, and R_{C} are as defined in formula (I).

In another embodiment, the present invention relates to a use for the manufacture of a medicament for treating epilepsy, migraine, or bipolar disorder in a mammal of a therapeutically effective amount of a compound of formula (I) wherein A is cycloalkyl wherein the cycloalkyl is optionally substituted with 1 or 2 alkyl groups; R₁ is NR₃R₄; R₃ is hydrogen; R₄ is alkyl; and R_{A}, R_{B}, and R_{C} are as defined in formula (I).

In another embodiment, the present invention relates to a use for the manufacture of a medicament for treating epilepsy, migraine, or bipolar disorder in a mammal of a therapeutically effective amount of a compound of formula (I) wherein A is cycloalkyl wherein the cycloalkyl is cyclohexyl optionally substituted with 1 or 2 alkyl groups; R₁ is NR₃R₄; R₃ is hydrogen; R₄ is alkyl; and R_{A}, R_{B}, and R_{C} are as defined in formula (I).

In another embodiment, the present invention relates to a use for the manufacture of a medicament for treating epilepsy, migraine, or bipolar disorder in a mammal of a therapeutically effective amount of a compound of formula (I) wherein A is cycloalkyl wherein the cycloalkyl is cyclohexyl; R₁ is NR₃R₄; R₃ is hydrogen; R₄ is alkyl, wherein a preferred alkyl group is methyl; and R_{A}, R_{B}, and R_{C} are hydrogen.

In another embodiment, the present invention relates to a use for the manufacture of a medicament for treating epilepsy, migraine, or bipolar disorder in a mammal of a therapeutically effective amount of a compound of formula (I) wherein A is cycloalkyl wherein the cycloalkyl is optionally substituted with 1 or 2 alkyl groups; R₁ is NR₃R₄; R₃ is hydrogen; R₄ is hydrogen or (NR₅R₆)carbonylalkyl; R₅ and R₆ are hydrogen; and R_{A}, R_{B}, and R_{C} are as defined in formula (I).

In another embodiment, the present invention relates to a use for the manufacture of a medicament for treating epilepsy, migraine, or bipolar disorder in a mammal of a therapeutically effective amount of a compound of formula (I) wherein A is cycloalkyl wherein the cycloalkyl is cyclohexane, cycloheptane, cyclooctane, cyclopentane, bicyclo[3.1.1]heptane, or bicyclo[2.2.1]heptane, wherein the cycloalkyl is optionally substituted with 1 or 2 alkyl groups; R₁ is NR₃R₄; R₃ is hydrogen; R₄ is hydrogen or (NR₅R₆)carbonylalkyl; R₅ and R₆ are hydrogen; R_{A} is hydrogen; and R_{B} and R_{C} are independently hydrogen or an alkyl group, wherein the preferred alkyl group is methyl.

In another embodiment, the present invention relates to a use for the manufacture of a medicament for treating epilepsy, migraine, or bipolar disorder in a mammal of a therapeutically effective amount of a compound of formula (I) wherein A is bicycloalkyl; R₁ is NR₃R₄; and R₃, R₄, R_{A}, R_{B}, and R_{C} are as defined in formula (I).

In another embodiment, the present invention relates to a use for the manufacture of a medicament for treating epilepsy, migraine, or bipolar disorder in a mammal of a therapeutically effective amount of a compound of formula (I) wherein A is bicycloalkyl wherein the bicycloalkyl is bicyclo[4.1.0]heptane, octahydro-1H-4,7-methanoindene, bicyclo[3.2.0]heptane, or octahydropentalene, wherein the bicycloalkyl is optionally substituted with 1 or 2 alkyl groups; R₁ is NR₃R₄; R₃ is hydrogen; R₄ is independently hydrogen or (NR₅R₆)carbonylalkyl; R_{A}, R₅ and R₆ are hydrogen; R_{B} and R_{C} are independently hydrogen or an alkyl group, wherein the preferred alkyl group is methyl.

In another embodiment, the present invention relates to a use for the manufacture of a medicament for treating psychiatric disorders, pain, or movement disorders, in a mammal of a therapeutically effective amount of a compound of formula (I).

In another embodiment, the present invention relates to a use for the manufacture of a medicament for providing neuroprotection in a mammal of a therapeutically effective amount of a compound of formula (I).

Representative compounds of formula (I) include, but are not limted to:
(1S,3S,5S,7R)-3,8,8-trimethyltricyclo[5.1.0.0^{3,5}]octane-4-carboxylic acid;
(1S,3S,4R,7R)-3,8,8-trimethyltricyclo[5.1.0.0^{3,5}]octane-4-carboxylic acid;
(1S,3S,4S,7R)-3,8,8-trimethyltricyclo[5.1.0.0^{3,5}]octane-4-carboxamide;
(1S,3S,4S,7R)-N-(2-amino-2-oxoethyl)-3,8,8trimethyltricyclo[5.1.0.0^{3,5}]octane-4-carboxamide;
(exo) (1R,6S)-bicyclo[4.1.0]heptane-7-carboxamide;
(exo) (1R,6S)-N-(2-amino-2-oxoethyl)bicyclo[4.1.0]heptane-7-carboxamide;
3-methylbicyclo[4.1.0]heptane-7-carboxylic acid;
3-methylbicyclo[4.1.0]heptane-7-carboxamide;
N-(2-amino-2-oxoethyl)-3-methylbicyclo[4.1.0]heptane-7-carboxamide;
(exo) (1R,2R,4S,5S)-tricyclo[3.2.1.0^{2,4}]octane-3-carboxylic acid;
(exo) (1R,2R,4S,5S)-tricyclo[3.2.1.0^{2,4}]octane-3-carboxamide;
(exo) (1R,2R,4S,5S)-N-(2-amino-2-oxoethyl)tricyclo[3.2.1.0^{2,4}]octane-3-carboxamide;
2,4-dimethylbicyclo[4.1.0]heptane-7-carboxylic acid;
2,4-dimethylbicyclo[4.1.0]heptane-7-carboxamide;
N-(2-amino-2-oxoethyl)-2,4-dimethylbicyclo[4.1.0]heptane-7-carboxamide;
(trans) 2,4-dimethylbicyclo[4.1.0]heptane-7-carboxylic acid;
2,4-dimethylbicyclo[4.1.0]heptane-7-carboxamide;
(1S,2S,4S,6R,7S)-N-(2-amino-2-oxoethyl)-2,4-dimethylbicyclo[4.1.0]heptane-7-carboxamide;
(exo) (1aR,2R,2aS,5aR,6S,6aS)-decahydro-2,6-methanocyclopropa[f]indene-1-carboxylic acid;
(exo) (1aR,2R,2aS,5aR,6S,6aS)-decahydro-2,6-methanocyclopropa[f]indene-1-carboxamide;
(exo) (1aR,2R,2aS,5aR,6S,6aS)-N-(2-amino-2-oxoethyl)decahydro-2,6-methanocyclopropa[f]indene-1-carboxamide;
(1R,5S)-tricyclo[3.3.0.0^{2,4}]oct-2(4)-ene-3-carboxylic acid;
(1R,5S)-tricyclo[3.3.0.0^{2,4}]oct-2(4)-ene-3-carboxamide;
(1R,5S)-N-(2-amino-2-oxoethyl)tricyclo[3.3.0.0^{2,4}]oct-2(4)-ene-3-carboxamide;
(2S,5R)-2-isopropyl-5-methylbicyclo[4.1.0]heptane-7-carboxylic acid;
(2S,5R)-2-isopropyl-5-methylbicyclo[4.1.0]heptane-7-carboxamide;
(25,5R)-N-(2-amino-2-oxoethyl)-2-isopropyl-5-methylbicyclo[4.1.0]heptane-7-carboxamide;
octahydro-1H-cyclopropa[a]pentalene-1-carboxylic acid;
octahydro-1H-cyclopropa[a]pentalene-1-carboxamide;
N-(2-amino-2-oxoethyl)octahydro-1H-cyclopropa[a]pentalene-1-carboxamide;
(endo) bicyclo[6.1.0]nonane-9-carboxylic acid;
(exo) bicyclo[6.1.0]nonane-9-carboxylic acid;
(endo) bicyclo[6.1.0]nonane-9-carboxamide;
(endo) N-(2-amino-2-oxoethyl)bicyclo[6.1.0]nonane-9-carboxamide;
(exo) bicyclo[6.1.0]nonane-9-carboxamide;
(exo) N-(2-amino-2-oxoethyl)bicyclo[6.1.0]nonane-9-carboxamide;
2,7,7-trimethyltricyclo[4.1.1.0^{2,4}]octane-3-carboxylic acid;
2,7,7-trimethyltricyclo [4.1.1.0^{2,4}]octane-3-carboxamide;
N-(2-amino-2-oxoethyl)-2,7,7-trimethyltricyclo[4.1.1.0^{2,4}]octane-3-carboxamide;
1-methylbicyclo[4.1.0]heptane-7-carboxylic acid;
1-methylbicyclo[4.1.0]heptane-7-carboxamide;
N-(2-amino-2-oxoethyl)-1-methylbicylo[4.1.0]heptane-7-carboxamide;
(exo) bicyclo[5.1.0]octane-8-carboxamide;
(exo) N-(2-amino-2-oxoethyl)bicyclo[5.1.0]octane-8-carboxamide;
(exo) bicyclo[3.1.0]hexane-6-carboxylic acid;
bicyclo[3.1.0]hexane-6-carboxamide;
(exo) N-(2-amino-2-oxoethyl)bicyclo[3.1.0]hexane-6-carboxamide;
4,7,7-trimethyltricyclo[4.1.1.0^{2,4}]octane-3-carboxylic acid;
4,7,7-trimethyltricyclo [4.1.1.0^{2,4}]octane-3-carboxamide;
N-(2-amino-2-oxoethyl)-4,7,7-trimethyltricyclo[4.1.1.0^{2,4}]octane-3-carboxamide;
3-tert-butylbicyclo[4.1.0]heptane-7-carboxylic acid;
3-tert-butylbicyclo[4.1.0]heptane-7-carboxamide;
N-(2-amino-2-oxoethyl)-3-tert-butylbicyclo[4.1.0]heptane-7-carboxamide;
(1R,2R,4R,7R)-4,8,8-trimethyltricyclo[5.1.0.0^{2,4}]octane-3-carboxylic acid;
(1R,2R,4R,7R)-4,8,8-trimethyltricyclo[5.1.0.0^{2,4}]octane-3-carboxamide;
(1R,2R,4R,7R)N-(2-amino-2-oxoethyl)-4,8,8-trimethyltricyclo[5.1.0.0^{2,4}]octane-3-carboxamide;
1-methylbicyclo[3.1.0]hexane-6-carboxylic acid;
1-methylbicyclo[3.1.0]hexane-6-carboxamide;
N-(2-amino-2-oxoethyl)-1-methylbicyclo[3.1.0]hexane-6-carboxamide;
1,5-dimethylbicyclo[4.1.0]heptane-7-carboxylic acid;
1,5-dimethylbicyclo[4.1.0]heptane-7-carboxamide; and
N-(2-amino-2-oxoethyl)-1,5-dimethylbicyclo[4.1.0]heptane-7-carboxamide; or a pharmaceutically acceptable prodrug thereof.

In another embodiment, the present invention relates to compounds of formula (II) 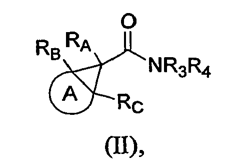 wherein
A is cycloalkyl or bicycloalkyl;
R_{A}, R_{B}, and R_{C} are independently hydrogen or alkyl;
R₃ is hydrogen or alkyl;
R₄ is alkenyl, alkynyl, alkoxycarbonylalkyl, carboxyalkyl, cycloalkyl, cycloalkylalkyl, heterocyclealkyl, hydroxyalkyl, (NR₅R₆)alkyl, (NR₅R₆)carbonylalkyl, or 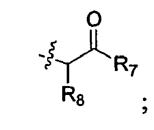 or
R₃ and R₄ taken together with the nitrogen atom to which they are attached form a heterocycle wherein the heterocycle is azepanyl, azetidinyl, aziridinyl, morpholinyl, piperazinyl, piperidinyl, pyrrolidinyl, or thiomorpholinyl;
R₅ and R₆ are independently hydrogen, alkenyl, alkyl, alkynyl, alkoxycarbonylalkyl, aryl, arylalkyl, cycloalkyl, cycloalkylalkyl, heterocycle, heterocyclealkyl, or hydroxyalkyl;
R₇ is alkoxy, alkyl, hydroxy, or NR₅R₆;
R₈ is alkenyl, alkoxyalkyl, alkoxycarbonylalkyl, alkylthioalkyl, alkynyl, aryl, arylalkyl, carboxyalkyl, cycloalkyl, cycloalkylalkyl, heterocycle, heterocyclealkyl, hydroxyalkyl, mercaptoalkyl, (NR₅R₆)alkyl, (NR₅R₆)carbonylalkyl, or -(CH₂)ₙNHC(=NH)₂; and
n is an integer from 1 to 6.

In another embodiment, the present invention relates to compounds of formula (II) wherein A is cycloalkyl; R₁ is NR₃R₄; and R₃, R₄, R_{A}, R_{B}, and R_{C} are as defined in formula (II).

In another embodiment, the present invention relates to compounds of formula (II) wherein A is cycloalkyl wherein the cycloalkyl is optionally substituted with 1 or 2 alkyl groups; R₃ is hydrogen; R₄ is (NR₅R₆)carbonylalkyl; R₅ and R₆ are hydrogen; and R_{A}, R_{B}, and R_{C} are as defined in formula (II).

In another embodiment, the present invention relates to compounds of formula (II) wherein A is cycloalkyl wherein the cycloalkyl is cyclohexane, cycloheptane, cyclooctane, cyclopentane, bicyclo[3.1.1]heptane, or bicyclo[2.2.1]heptane, wherein the cycloalkyl is optionally substituted with 1 or 2 alkyl groups; R₃ is hydrogen; R₄ is (NR₅R₆)carbonylalkyl; R₅ and R₆ are hydrogen; R_{A} is hydrogen; and R_{B} and R_{C} are independently hydrogen or an alkyl group, wherein a preferred alkyl group is methyl.

In another embodiment, the present invention relates to compounds of formula (II) wherein A is cycloalkyl wherein the cycloalkyl is cyclohexane, cycloheptane, cyclooctane, cyclopentane, bicyclo[3.1.1]heptane, or bicyclo[2.2.1]heptane, wherein the cycloalkyl is optionally substituted with 1 or 2 alkyl groups; R₃ is hydrogen; R₄ is (NR₅R₆)carbonylalkyl wherein the (NR₅R₆)carbonylalkyl is 2-amino-2-oxoethyl; R_{A} is hydrogen; and R_{B} and R_{C} are independently hydrogen or an alkyl group, wherein a preferred alkyl group is methyl.

In another embodiment, the present invention relates to compounds of formula (II) wherein A is bicycloalkyl; and R₃, R₄, R_{A}, R_{B}, and R_{C} are as defined in formula (II).

In another embodiment, the present invention relates to compounds of formula (II) wherein A is bicycloalkyl wherein the bicycloalkyl is optionally substituted with 1 or 2 alkyl groups ; R₃ is hydrogen; R₄ is (R₅R₆)carbonylalkyl; R₅ and R₆ are hydrogen; and R_{A}, R_{B}, and R_{C} are as defined in formula (II).

Representative compounds of formula (II) include, but are not limited to:
(exo) (1R,6S)-N-(2-amino-2-oxoethyl)bicyclo[4.1.0]heptane-7-carboxamide;
N-(2-amino-2-oxoethyl)-3-methylbicyclo[4.1.0]heptane-7-carboxamide;
(exo) (1R,2R,4S,5S)-N-(2-amino-2-oxoethyl)tricyclo[3.2.1.0^{2,4}]octane-3-carboxamide;
N-(2-amino-2-oxoethyl)-2,4-dimethylbicyclo[4.1.0]heptane-7-carboxamide;
(1S,2S,4S,6R,7S)-N-(2-amino-2-oxoethyl)-2,4-dimethylbicyclo[4.1.0]heptane-7-carboxamide;
(2S,5R)-N-(2-amino-2-oxoethyl)-2-isopropyl-5-methylbicyclo[4.1.0]heptane-7-carboxamide;
(endo) N-(2-amino-2-oxoethyl)bicyclo[6.1.0]nonane-9-carboxamide;
(exo) N-(2-amino-2-oxoethyl)bicyclo[6.1.0]nonane-9-carboxamide;
N-(2-amino-2-oxoethyl)-2,7,7-trimethyltricyclo[4.1.1.0^{2,4}]octane-3-carboxamide;
N-(2-amino-2-oxoethyl)-1-methylbicylo[4.1.0]heptane-7-carboxamide;
(exo) N-(2-amino-2-oxoethyl)bicyclo[5.1.0]octane-8-carboxamide;
(exo) N-(2-amino-2-oxoethyl)bicyclo[3.1.0]hexane-6-carboxamide;
N-(2-amino-2-oxoethyl)-4,7,7-trimethyltricyclo [4.1.1.0^{2,4}]octane-3-carboxamide;
N-(2-amino-2-oxoethyl)-3-tert-butylbicyclo[4.1.0]heptane-7-carboxamide;
N-(2-amino-2-oxoethyl)-1-methylbicyclo[3.1.0]hexane-6-carboxamide;
N-(2-amino-2-oxoethyl)-l,5-dimethylbicyclo[4.1.0]heptane-7-carboxamide;
(1S,3S,4S,7R)-N-(2-amino-2-oxoethyl)-3,8,8-trimethyltricyclo[5.1.0.0^{3,5}]octane-4-carboxamide;
(exo) (1aR,2R,2aS,5aR,6S,6aS)-N-(2-amino-2-oxoethyl)decahydro-2,6-methanocyclopropa[f]indene-1-carboxamide;
(1R,5S)-N-(2-amino-2-oxoethyl)tricyclo[3.3.0.0^{2,4}]oct-2(4)-ene-3-carboxamide;
N-(2-amino-2-oxoethyl)octahydro-1H-cyctopropa[a]pentalene-1-carboxamide; and
(1R,2R,4R,7R)-N-(2-amino-2-oxoethyl)-4,8,8-trimethyltricyclo[5.1.0.0^{2,4}]octane-3-carboxamide.

### Definition of Terms

As used throughout this specification and the appended claims, the following terms have the following meanings:

The term "alkenyl" as used herein, means a straight or branched chain hydrocarbon containing from 2 to 10 carbons and containing at least one carbon-carbon double bond formed by the removal of two hydrogens. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, and 3-decenyl.

The term "alkoxy" as used herein, means an alkyl group, as defined herein, appended to the parent molecular moiety through an oxygen atom. Representative examples of alkoxy include, but are not limited to, methoxy, ethoxy, propoxy, 2-propoxy, butoxy, tert-butoxy, pentyloxy, and hexyloxy.

The term "alkoxyalkoxy" as used herein, means an alkoxy group, as defined herein, appended to the parent molecular moiety through another alkoxy group, as defined herein. Representative examples of alkoxyalkoxy include, but are not limited to, tert-butoxymethoxy, 2-ethoxyethoxy, 2-methoxyethoxy, and methoxymethoxy.

The term "alkoxyalkyl" as used herein, means an alkoxy group, as defined herein, appended to the parent molecular moiety through an alkyl group, as defined herein. Representative examples of alkoxyalkyl include, but are not limited to, tert-butoxymethyl, 2-ethoxyethyl, 2-methoxyethyl, and methoxymethyl.

The term "alkoxycarbonyl" as used herein, means an alkoxy group, as defined herein, appended to the parent molecular moiety through a carbonyl group, as defined herein. Representative examples of alkoxycarbonyl include, but are not limited to, methoxycarbonyl, ethoxycarbonyl, and tert-butoxycarbonyl.

The term "alkoxycarbonylalkyl" as used herein, means an alkoxycarbonyl group, as defined herein, appended to the parent molecular moiety through an alkyl group, as defined herein. Representative examples of alkoxycarbonylalkyl include, but are not limited to, 3-methoxycarbonylpropyl, 4-ethoxycarbonylbutyl, and 2-tert-butoxycarbonylethyl.

The term "alkoxysulfonyl" as used herein, means an alkoxy group, as defined herein, appended appended to the parent molecular moiety through a sulfonyl group, as defined herein. Representative examples of alkoxysulfonyl include, but are not limited to, methoxysulfonyl, ethoxysulfonyl and propoxysulfonyl.

The term "alkyl" as used herein, means a straight or branched chain hydrocarbon containing from 1 to 10 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, and n-decyl.

The term "alkylcarbonyl" as used herein, means an alkyl group, as defined herein, appended to the parent molecular moiety through a carbonyl group, as defined herein. Representative examples of alkylcarbonyl include, but are not limited to, acetyl, 1-oxopropyl, 2,2-dimethyl-1-oxopropyl, 1-oxobutyl, and 1-oxopentyl.

The term "alkylcarbonylalkyl" as used herein, means an alkylcarbonyl group, as defmed herein, appended to the parent molecular moiety through an alkyl group, as defined herein. Representative examples of alkylcarbonylalkyl include, but are not limited to, 2-oxopropyl, 3,3-dimethyl-2-oxopropyl, 3-oxobutyl, and 3-oxopentyl.

The term "alkylcarbonyloxy" as used herein, means an alkylcarbonyl group, as defined herein, appended to the parent molecular moiety through an oxygen atom. Representative examples of alkylcarbonyloxy include, but are not limited to, acetyloxy, ethylcarbonyloxy, and tert-butylcarbonyloxy.

The term "alkylene" means a divalent group derived from a straight or branched chain hydrocarbon of from 1 to 10 carbon atoms. Representative examples of alkylene include, but are not limited to, -CH₂-, -CH(CH₃)-, -C(CH₃)₂-, -CH₂CH₂-, -CH₂CH₂CH₂-, -CH₂CH₂CH₂CH₂-, and -CH₂CH(CH₃)CH₂-.

The term "alkylsulfonyl" as used herein, means an alkyl group, as defined herein, appended to the parent molecular moiety through a sulfonyl group, as defined herein. Representative examples of alkylsulfonyl include; but are not limited to, methylsulfonyl and ethylsulfonyl.

The term "alkylthio" as used herein, means an alkyl group, as defined herein, appended to the parent molecular moiety through a sulfur atom. Representative examples of alkylthio include, but are not limited, methylthio, ethylthio, tert-butylthio, and hexylthio.

The term "alkylthioalkyl" as used herein, means an alkylthio group, as defined herein, appended to the parent molecular moiety through an alkyl group, as defined herein. Representative examples of alkylthioalkyl include, but are not limited, methylthiomethyl and 2-(ethylthio)ethyl.

The term "alkynyl" as used herein, means a straight or branched chain hydrocarbon group containing from 2 to 10 carbon atoms and containing at least one carbon-carbon triple bond. Representative examples of alkynyl include, but are not limited, to acetylenyl, 1-propynyl, 2-propynyl, 3-butynyl, 2-pentynyl, and 1-butynyl.

The term "aryl" as used herein, means a monocyclic-ring system, or a bicyclic- or a tricyclic-fused ring system wherein one or more of the fused rings are aromatic. Representative examples of aryl include, but are not limited to, anthracenyl, azulenyl, fluorenyl, 2,3-dihydroindenyl, indenyl, naphthyl, phenyl, and tetrahydronaphthyl.

The aryl groups of this invention can be optionally substituted with 1, 2, or 3 substituents independently selected from the group consisting of alkenyl, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfonyl, alkylthio, alkylthioalkyl, alkynyl, carboxy, carboxyalkyl, cyano, cyanoalkyl, ethylenedioxy, formyl, haloalkoxy, haloalkyl, halogen, hydroxy, hydroxyalkyl, mercapto, methylenedioxy, nitro, NR_{D}R_{E}, and (NR_{D}R_{E})carbonyl.

The term "arylalkyl" as used herein, means an aryl group, as defined herein, appended to the parent molecular moiety through an alkyl group, as defined herein. Representative examples of arylalkyl include, but are not limited to, benzyl, 2-phenylethyl, 3-phenylpropyl, and 2-naphth-2-ylethyl.

The term "bicycloalkyl" as used herein, means a cycloalkyl group, as defined herein, fused to another cycloalkyl goup, as defined herein. Representative examples of bicycloalkyl, include, but are not limited to, bicyclo[3.2.0]heptane, bicyclo[4.1.0]heptane, bicyclo[4.2.0]octane, decahydronaphthalenyl, octahydro-1H-indenyl, octahydropentalenyl, and octahydro-1H-4,7-methanoindene.

The bicycloalkyl groups of the present invention are substituted with 0, 1, 2, 3, or 4 substituents selected from the group consisting of alkenyl, alkoxy, alkoxycarbonyl, alkyl, alkylcarbonyl, alkylthio, alkynyl, carboxy, cyano, formyl, haloalkoxy, haloalkyl, halogen, hydroxy, oxo, mercapto, -NR_{D}R_{E}, and (NR_{D}R_{E})carbonyl.

The term "carbonyl" as used herein, means a-C(O)- group.

The term "carboxy" as used herein, means a -CO₂H group.

The term "carboxyalkyl" as used herein, means a carboxy group, as defined herein, appended to the parent molecular moiety through an alkyl group, as defined herein. Representative examples of carboxyalkyl include, but are not limited to, carboxymethyl, 2-carboxyethyl, and 3-carboxypropyl.

The term "cyano" as used herein, means a-CN group.

The term "cyanoalkyl" as used herein, means a cyano group, as defined herein, appended to the parent molecular moiety through an alkyl group, as defined herein. Representative examples of cyanoalkyl include, but are not limited to, cyanomethyl, 2-cyanoethyl, and 3-cyanopropyl.

The term "cycloalkyl" as used herein, means a monocyclic ring system or a bridged monocyclic ring system. Monocyclic ring systems are exemplified by a saturated cyclic hydrocarbon group containing from 3 to 8 carbon atoms. Examples of monocyclic ring systems include cyclopropyl, cyclobutyl, cyclopentyl, cyclohexyl, cycloheptyl, and cyclooctyl. Bridged monocyclic ring systems are exemplified by a monocyclic ring system in which two non-adjacent carbon atoms of the monocyclic ring are linked by an alkylene group, as defined herein. representative examples of bridged monocyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane.

The cycoalkyl groups of the present invention are optionally substituted with 1, 2, 3, or 4 substituents selected from the group consisting of alkenyl, alkoxy, alkoxycarbonyl, alkyl, alkylcarbonyl, alkylthio, alkynyl, carboxy, cyano, formyl, haloalkoxy, haloalkyl, halogen, hydroxy, oxo, mercapto, NR_{D}R_{E}, and (NR_{D}R_{E})carbonyl.

The term "cycloalkylalkyl" as used herein, means a cycloalkyl group, as defined herein, appended to the parent molecular moiety through an alkyl group, as defined herein. Representative examples of cycloalkylalkyl include, but are not limited to, cyclopropylmethyl, 2-cyclobutylethyl, cyclopentylmethyl, cyclohexylmethyl, and 4-cycloheptylbutyl.

The term "ethylenedioxy" as used herein, means a -O(CH₂)₂O- group wherein the oxygen atoms of the ethylenedioxy group are attached to the parent molecular moiety through one carbon atom forming a 5 membered ring or the oxygen atoms of the ethylenedioxy group are attached to the parent molecular moiety through two adjacent carbon atoms forming a six membered ring.

The term "formyl" as used herein, means a-C(O)H group.

The term "halo" or "halogen" as used herein, means -Cl,-Br, -I or -F.

The term "haloalkoxy" as used herein, means at least one halogen, as defined herein, appended to the parent molecular moiety through an alkoxy group, as defined herein. Representative examples of haloalkoxy include, but are not limited to, chloromethoxy, 2-fluoroethoxy, trifluoromethoxy, and pentafluoroethoxy.

The term "haloalkyl" as used herein, means at least one halogen, as defined herein, appended to the parent molecular moiety through an alkyl group, as defined herein. Representative examples of haloalkyl include, but are not limited to, chloromethyl, 2-fluoroethyl, trifluoromethyl, pentafluoroethyl, and 2-chloro-3-fluoropentyl.

The term "heterocycle" or "heterocyclic" as used herein, means a monocyclic, bicyclic, or tricyclic ring system. Monocyclic ring systems are exemplified by any 3- or 4-membered ring containing a heteroatom independently selected from the group consisting of oxygen, nitrogen and sulfur; or a 5-, 6- or 7-membered ring containing one, two or three heteroatoms wherein the heteroatoms are independently selected from the group consisting of nitrogen, oxygen and sulfur. The 5-membered ring has from 0-2 double bonds and the 6- and 7-membered ring have from 0-3 double bonds. Representative examples of monocyclic ring systems include, but are not limited to, azetidinyl, azepanyl, aziridinyl, diazepinyl, 1,3-dioxolanyl, dioxanyl, dithianyl, furyl, imidazolyl, imidazolinyl, imidazolidinyl, isothiazolyl, isothiazolinyl, isothiazolidinyl, isoxazolyl, isoxazolinyl, isoxazolidinyl, morpholinyl, oxadiazolyl, oxadiazolinyl, oxadiazolidinyl, oxazolyl, oxazolinyl, oxazolidinyl, piperazinyl, piperidinyl, pyranyl, pyrazinyl, pyrazolyl, pyrazolinyl, pyrazolidinyl, pyridinyl, pyrimidinyl, pyridazinyl, pyrrolyl, pyrrolinyl, pyrrolidinyl, tetrahydrofuranyl, tetrahydrothienyl, tetrazinyl, tetrazolyl, thiadiazolyl, thiadiazolinyl, thiadiazolidinyl, thiazolyl, thiazolinyl, thiazolidinyl, thienyl, thiomorpholinyl, 1,1-dioxidothiomorpholinyl (thiomorpholine sulfone), thiopyranyl, triazinyl, triazolyl, and trithianyl. Bicyclic ring systems are exemplified by any of the above monocyclic ring systems fused to an aryl group as defined herein, a cycloalkyl group as defined herein, or another monocyclic ring system. Representative examples of bicyclic ring systems include but are not limited to, for example, benzimidazolyl, benzodioxinyl, benzothiazolyl, benzothienyl, benzotriazolyl, benzoxazolyl, benzofuranyl, benzopyranyl, benzothiopyranyl, cinnolinyl, indazolyl, indolyl, 2,3-dihydroindolyl, indolizinyl, naphthyridinyl, isobenzofuranyl, isobenzothienyl, isoindolyl, isoquinolinyl, phthalazinyl, pyranopyridinyl, quinolinyl, quinolizinyl, quinoxalinyl, quinazolinyl, tetrahydroisoquinolinyl, tetrahydroquinolinyl, and thiopyranopyridinyl. Tricyclic rings systems are exemplified by any of the above bicyclic ring systems fused to an aryl group as defined herein, a cycloalkyl group as defined herein, or a monocyclic ring system. Representative examples of tricyclic ring systems include, but are not limited to, acridinyl, carbazolyl, carbolinyl, dibenzo[b,d]furanyl, dibenzo[b,d]thienyl, naphtho[2,3-b]furan, naphtho[2,3-b]thienyl, phenazinyl, phenothiazinyl, phenoxazinyl, thianthrenyl, thioxanthenyl and xanthenyl.

The heterocycles of this invention are optionally substituted with 1, 2,or 3 substituents independently selected from the group consisting of alkenyl, alkoxy, alkoxyalkoxy, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylthio, alkylthioalkyl, alkynyl, carboxy, carboxyalkyl, cyano, cyanoalkyl, ethylenedioxy, formyl, haloalkoxy, haloalkyl, halogen, hydroxy, hydroxyalkyl, mercapto, methylenedioxy, nitro, oxo, -NR_{D}R_{E}, and (NR_{D}R_{E})carbonyl.

The term "heterocyclealkyl" as used herein, means a heterocycle, as defined herein, appended to the parent molecular moiety through an alkyl group, as defined herein. Representative examples of heterocyclealkyl include, but are not limited to, pyridin-3-ylmethyl and 2-pyrimidin-2-ylpropyl.

The term "hydroxy" as used herein, means an -OH group.

The term "hydroxyalkyl" as used herein, means at least one hydroxy group, as defined herein, is appended to the parent molecular moiety through an alkyl group, as defined herein. Representative examples of hydroxyalkyl include, but are not limited to, hydroxymethyl, 2-hydroxyethyl, 2-hydroxypropyl, 3-hydroxypropyl, 2,3-dihydroxypentyl, and 2-ethyl-4-hydroxyheptyl.

The term "mercapto" as used herein, means a -SH group.

The term "mercaptoalkyl" as used herein, means a mercapto group, as defined herein, appended to the parent molecular moiety through an alkyl group, as defined herein. Representative examples of mercaptoalkyl include, but are not limited to, 2-mercaptoethyl and 3-mercaptopropyl.

The term "methylenedioxy" as used herein, means a -OCH₂O- group wherein the oxygen atoms of the methylenedioxy are attached to the parent molecular moiety through two adjacent carbon atoms.

The term "nitro" as used herein, means a -NO₂ group.

The term "-NR_{D}R_{E}" as used herein, means two groups, R_{D} and R_{E}, which are appended to the parent molecular moiety through a nitrogen atom. R_{D} and R_{E} are each independently selected from the group consisting of hydrogen, alkenyl, alkoxycarbonyl, alkoxysulfonyl, alkyl, alkylcarbonyl, alkylsulfonyl, alkynyl, and formyl. Representative examples of -NR_{D}R_{E} include, but are not limited to, amino, acetylamino, methylamino, dimethylamino, ethylamino, ethylmethylamino, benzylamino, methoxysulfonylamino, methylsulfonylamino, ethoxycarbonylamino, and tert-butoxycarbonylamino.

The term "(NR_{D}R_{E})carbonyl" as used herein, means a -NR_{D}R_{E} group, as defined herein, appended to the parent molecular moiety through a carbonyl group, as defined herein. Representative examples of (NR_{D}R_{E})carbonyl include, but are not limited to, aminocarbonyl, (methylamino)carbonyl, (dimethylamino)carbonyl and (ethylmethylamino)carbonyl.

The term "(NR₅R₆)carbonyl" as used herein, means a -NR₅R₆ group, as defined herein, appended to the parent molecular moiety through a carbonyl group, as defined herein. Representative examples of (NR₅R₆)carbonyl include, but are not limited to, aminocarbonyl, (methylamino)carbonyl, (dimethylamino)carbonyl, and (ethylmethylamino)carbonyl.

The term "(NR₅R₆)carbonylalkyl" as used herein, means a (NR₅R₆)carbonyl group, as defined herein, appended to the parent molecular moiety through an alkyl group, as defined herein. Representative examples of (NR₅R₆)carbonylalkyl include, but are not limited to, 2-amino-2-oxoethyl, 2-methylamino-2-oxoethyl, and 2-dimethylamino-2-oxoethyl.

The term "oxo" as used herein, means a =O moiety.

The term "sulfonyl" as used herein, means a -SO₂- group.

Compounds of the present invention were named by ACD/ChemSketch version 5.0 (developed by Advanced Chemistry Development, Inc., Toronto, ON, Canada) or were given names consistent with ACD nomenclature.

Compounds of the present invention can exist as stereoisomers, wherein asymmetric or chiral centers are present. Stereoisomers are designated "R" or "S," depending on the configuration of substituents around the chiral carbon atom. The terms "R" and "S" used herein are configurations as defined in IUPAC 1974 Recommendations for Section E, Fundamental Stereochemistry, Pure Appl. Chem., (1976), 45: 13-30. The present invention contemplates various stereoisomers and mixtures thereof and are specifically included within the scope of this invention. Stereoisomers include enantiomers, diastereomers, and mixtures of enantiomers or diastereomers. Individual stereoisomers of compounds of the present invention may be prepared synthetically from commercially available starting materials which contain asymmetric or chiral centers or by preparation of racemic mixtures followed by resolution, a technique well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) direct separation of the mixture of optical enantiomers on chiral chromatographic columns, or (3) formation of a diastereomeric salt followed by selective recrystallization of one of the diastereomeric salts.

### Abbreviations

Abbreviations which have been used in the descriptions of the schemes and the examples that follow are: DMSO for dimethylsulfoxide; EtOAc for ethyl acetate; EtOH for ethanol; and MeOH for methanol.

### Preparation of Compounds of The Invention

The compounds of the present invention can be prepared by a variety of synthetic routes. Representative procedures are shown in Scheme 1-2.

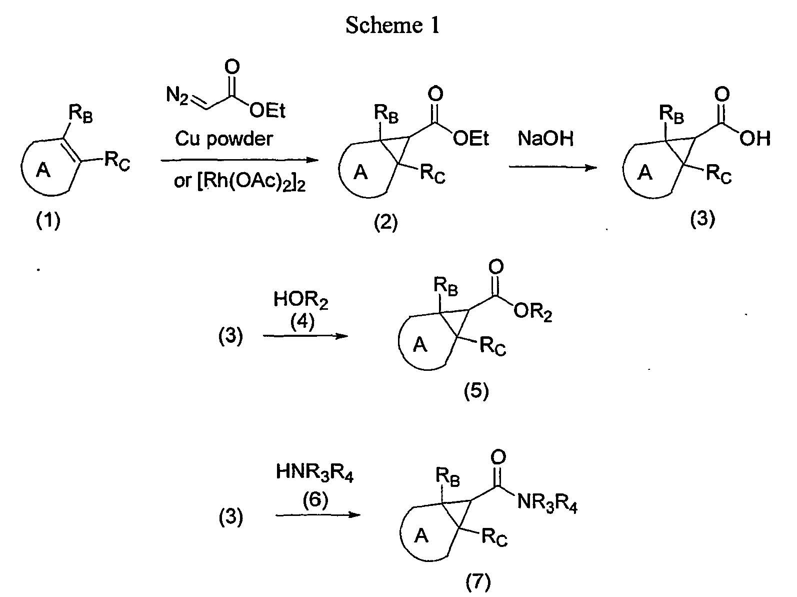

Compounds of the present invention, wherein A, R_{B}, R_{C}, R₂, R₃, and R₄ are as defined in formula (I), can be prepared as described in Scheme 1. Cyclic alkenes of general formula (1), purchased or prepared using methodology known to those of skill in the art, can be treated with ethyl diazoacetate and copper powder or rhodium(II) acetate dimmer to provide fused cyclopropane esters of general formula (2). Fused cyclopropane esters of general formula (2) can be saponified to provide acids of general formula (3). Acids of general formula (3) can be treated with thionyl chloride and an alcohol of general formula (4) to provide esters of general formula (5). Acids of general formula (3) can also be treated with amines of general formula (6) and a coupling reagent including, but not limited to, 1,1'-carbonyldiimidazole (CDI), 1,1'-thiocarbonyldiimidazole, 1,3-dicyclohexylcarbodiimide, 1-ethyl-3-[3-(dimethylamino)propyl]carbodiimide hydrochloride, or thionylchloride, to provide amides of general formula (7).

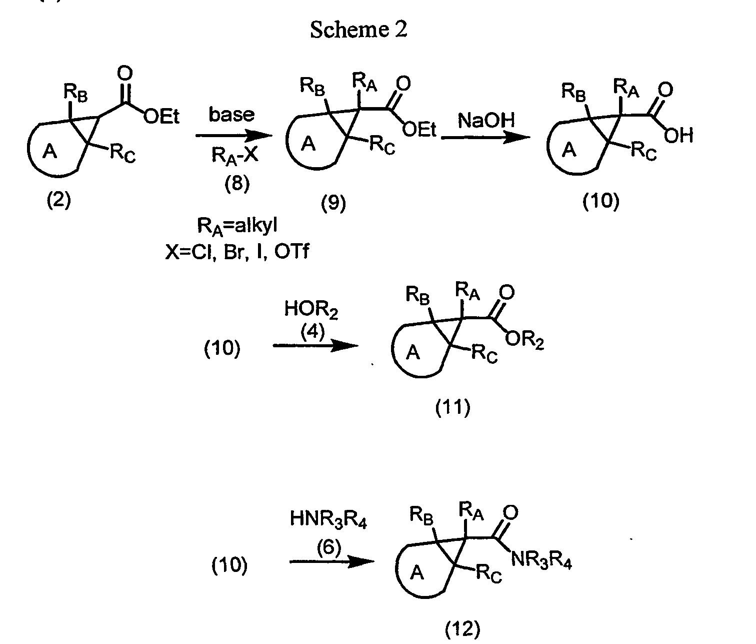

Fused cyclopropanes of general formula (10), (11) and (12), wherein A, R_{B}, R_{C}, R₂, R₃, and R₄ are as defined in formula (I) and R_{A} is alkyl as defined herein, can be prepared as described in Scheme 2. Fused cyclopropanes of general formula (2), prepared as described in Scheme 1, can be treated with a base, including but not limited to, lithium diisopropylamide, sodium bis(trimethylsilyl)amide, potassium bis(trimethylsilyl)amide, lithium bis(trimethylsilyl)amide, or sodium hydride and an alkyl halide or an alkyl trifluoromethanesulfonate of general formula (8), including but not limited to iodomethane in a solvent, including but not limited to, THF or DMF to provide esters of general formula (9). Esters of general formula (9) can be processed as described in Scheme 1 to provide fused cyclopropanes of general formula (10), (11), and (12).

### Example 1

### (1S,3S,4S,7R)-3,8,8-trimethyltricyclo[5.1.0.0^{3,5}]octane-4-carboxylic acid

### Example 1A

### ethyl (1S,3S,4R,7R)-3,8,8-trimethyltricyclo[5.1.0.0^{3,5}]octane-4-carboxylate

### ethyl (1S,3S,4S,7R)-3,8,8-trimethyltricyclo[5.1.0.0^{3,5}]octane-4-carboxylate

A mixture of copper powder (1.11 g, 17.5 mmol) and (1S)(+)-3-carene (69.7 g, 512 mmol, purchased from Aldrich) was warmed to 100 °C under an atmosphere of nitrogen. Ethyl diazoacetate (48.6 g, 426 mmol, purchased from Aldrich) was added over a 6-hour period using a syringe pump. The mixture was stirred at 100 °C for an additional hour, then cooled to ambient temperature and filtered through diatomaceous earth (50 mL EtOAc rinse). The filtrate was concentrated to afford an oil (104 g). A portion of this mixture (49 g) was purified by chromatography (silica gel, 10:90 EtOAc:hexane) to afford 29.5 g of a 4.4:1 mixture of diastereomers as determined by HPLC analysis: Zobax RX-C8 column, 5 µm, 4.6 x 250 mm; λ = 200 nm, flow rate 1.5 mL/minute, ambient temperature, eluted with 10→90% CH₃CN / 0.1 % H₃PO₄ gradient over 15 minutes then hold at 90% CH₃CN / 0.1 % H₃PO₄ for 5 minutes; retention times of and 15.2 minutes for the (1S,3S,4S,7R) diastereomer and 15.7 minutes for the (1S,3S,4R,7R) diastereomer.

### Example 1B

### (1S,3S,4S,7R)-3,8,8-trimethyltricyclo[5.1.0.^{3,5}]octane-4-carboxylic acid

The 4.4:1 mixture of the exo:endo diastereomers from Example 1A (29.5 g, 133 mmol) were added to a solution of NaOH (21.6 g, 540 mmol) in water (144 mL) and vigorously stirred at 100 °C for 15 hours. The solution was cooled to ambient temperature resulting in formation of a precipitate that was isolated by filtration (H₂O wash). The precipitate was diluted with H₂O (100 mL) and acidified by the addition of concentrated HCl (20 mL). The surry was filtered (H₂O wash) and the obtained solid was dried under reduced pressure at 45 °C to afford the title compound (11.9 g), which was determined to be diasteromerically pure by HPLC analysis: HPLC conditions as described in Example 1A, retention time of 12.0 minutes. A sample was recrystallized from H₂O/EtOH: mp 86.0 °C;¹H NMR (CDCl₃, 300 MHz) δ 0.35(d of t), 0.42(d of t), 0.76(s), 1.00(s), 1.16-1.21(m), 1.19(s), 1.33-1.38(t), 1.40-1.42(t), 1.44-1.47(m), 1.60(d), 2.04-2.13(m);¹³C NMR (CDCl₃, 100 MHz) δ 14.2, 15.0, 16.3, 16.4, 16.9, 19.2, 24.4, 24.8, 26.6, 27.4, 27.9, 180.2; MS m/z 195 (M+H)⁺.

### Example 2

### (1S,3S,4R,7R)-3,8,8-trimethyltricyclo[5.1.0.0^{3,5}]octane-4-carboxylic acid

The basic aqueous NaOH filtrate from Example 1B was extracted with heptane (2 x 70 mL), acidified with concentrated HCl, and extracted with CH₂Cl₂. The CHCl₂ extract was concentrated to give an oil that was slurried with heptane. The resulting precipitate was isolated by filtration (cold heptane rinse). The solid was dried under reduced pressure at 45 °C to afford 2.5 g of the title compound, which was diastereomerically pure by HPLC (conditions as described in Example 1A, retention time of 11.5 minutes. ¹H NMR (CDCl₃, 300 MHz) δ 0.43(tt), 0.79(s), 0.97(s), 1.04(t), 1.08(s), 1.32(d), 1.40(dd), 1.73(dd), 2.14(q), 2.18(q); ¹³C NMR (δ, CDCl₃) 14.2, 14.8, 16.2, 16.7, 18.3, 20.6, 20.9, 24.2, 27.5, 28.0, 177.7; MS m/z 194 (M+NH₄)⁺.

### Example 3

### (1S,3S,4S,7R)-3,8,8-trimethyltricyclo[5.1.0.0^{3,5}]octane-4-carboxamide

1,1'-Carbonyldiimidazole (1.69 g, 10.4 mmol, purchased from Aldrich) was added to a solution of the the product from Example 1B (1.92 g, 9.88 mmol) in EtOAc (20 mL) at room temperature. The mixture was stirred at room temperature for 1.5 hours then cooled to 0 °C and concentrated ammonium hydroxide (5 mL) was added. The mixture was stirred at room temperature for 14 hours then washed with H₂O (10 mL), 15% aqueous citric acid (10 mL), and H₂O (10 mL). The EtOAc extract was concentrated to afford the title compound (1.80 g, 94%) as a white solid. A sample was recrystrallized from heptane/EtOAc: mp 121.5-122.5 °C;¹H NMR (CDCl₃, 300 MHz) δ 0.31-0.35(dt), 0.36-0.44(dt), 0.77(s), 1.01(s), 1.14(s), 1.16-1.21(dd), 1.36-1.42(m), 1.98-2.10(m), 5.45(br s); ¹³C NMR (CDCl₃, 100 MHz) δ 14.6, 15.0, 16.2, 16.5, 16.9, 18.9, 24.0, 24.4, 24.5, 26.1, 28.0, 174.7;MS m/z 194 (M+H)⁺.

### Example 4

### (1S,3S,4S,7R)-N-(2-amino-2-oxoethyl)-3,8,8 trimethyltricyclo[5.1.0.0^{3,5}]octane-4-carboxamide

1,1'-Carbonyldiimidazole (1.69 g, 10.9 mmol, purchased from Alrdrich) was added to a solution of the product from Example 1B (2.00 g, 10.3 mmol) in EtOAc (20 mL) at room temperature. The mixture was stirred at room temperature for 1.5 hours then 2-aminoacetamide hydrochloride (1.20 g, 10.9 mmol, purchased from Aldrich) was added. The mixture was stirred at 75 °C for 8 hours then cooled to ambient temperature and washed with H₂O (10 mL), 15% aqueous citric acid (2 x 10 mL), and H₂O (10 mL). The EtOAc extract was concentrated to afford a solid that was recrystallized from EtOH/EtOAc to afford the title compound as a white solid: mp 169.2-169.7 °C; ¹H NMR (CDCl₃, 300 MHz) δ 0.31-0.36(dt), 0.38-0.44(dt), 0,.76(s), 1.01(s), 1.11(s), 1.15-1.19(dd), 1.35-1.45(m), 1.98-2.09(m), 3.99(d), 5.54(br s), 6.44(t), 6.48(br s); ¹³C NMR (CDCl₃, 100 MHz) δ 14.5, 15.0, 16.2, 16.5, 16.9, 19.0, 24.1, 24.3, 24.5, 26.5, 28.0, 43.3, 171.1, 173.1; MS m/z 251 (M+H)⁺.

### Example 5

### (exo) (1R,6S)-bicyclo[4.1.0]heptane-7-carboxamide

### Example 5A

### (exo) ethyl (1R,6S)-bicyclo[4.1.0]heptane-7-carboxylate

### (endo) ethyl (1R,6S)-bicyclo[4.1.0]heptane-7-carboxylate

A mixture of rhodium(II) acetate (0.25 g, 0.566 mmol, purchased from Aldrich) and cyclohexene (80 mL, 790 mmol, purchased from Aldrich) was purged with nitrogen. Ethyl diazoacetate (100 g, 876 mmol, purchased from Aldrich) was added at a rate of 1.5 mL/hour via a syringe pump. The mixture was filtered through a bed of diatomaceous earth (50 mL EtOAc rinse) to remove the catalyst. The filtrate was concentrated to a green oily residue (120 g) that was purified by reduced pressure distillation (1.5 mm Hg, 65-75 °C) to afford the title compound as a colorless oil (103 g, 84% yield, 3.6:1 mixture of the exo/endo-diastereomers, respectively).

### Example 5B

### (exo) (1R,6S)-bicyclo[4.1.0]heptane-7-carboxylic acid

### (endo) (1R,6S)-bicyclo[4.1.0]heptane-7-carboxylic acid

The title compounds were prepared as a 3.6:1 mixture of exo/endo-diastereomers by using the procedure described in Example 1B substituting the products from Example 5A for the product from Example 1A.

### Example 5C

### (exo) (1R,6S)-bicyclo[4.1.0]heptane-7-carboxamide

The title compound was prepared by using the procedure described in Example 3 substituting the products from Example 5B for the product from Example 1B. A 3.7:1 ratio of exo/endo-diastereomers was isolated (HPLC: Zorbax RX-C8 column, 5 µm, 4.6 x 250 mm; λ = 200 nm, flow rate 1.5 mL/minute, 35 °C, eluted with 10→90% CH₃CN / 0.1% H₃PO₄ gradient over 15 minutes then hold at 90% CH₃CN / 0.1% H₃PO₄ for 5 minutes; retention times of 7.24 minutes for the endo diastereomer and 7.05 minutes for the exo diastereomer. The mixture was washed with 1.5 M aqueous citric acid and recrystallized from EtOAc/hexane to afford the title compound (51% yield) which was determined to be diastereomerically pure by HPLC analysis. ¹H NMR (DMSO-d₆ 300 MHz) δ 1.18 (m), 1.28 (m), 1.32 (t), 1.44 (m), 1.66 (m), 1.88 (m), 6.02 (s), 6.94 (s); ¹³C NMR (CDCl₃, 100 MHz) δ 19.7, 20.5, 22.2, 26.1, 175.6; MS m/z 140 (M+H)⁺.

### Example 6

### (exo) (1R,6S)-N-(2-amino-2-oxoethyl)bicyclo[4.1.0]heptane-7-carboxamide

The title compound was prepared by using the procedure described in Example 4 substituting the products from Example 5B for the product from Example 1B. A 3.7:1 ratio of exo/endo-diastereomers was isolated (HPLC: Zobax SB-C8 column, 3.5 µm, 4.6 x 250 mm; λ = 200 nm, flow rate 1.5 mL/minute, 35 °C, eluted with 10→90% CH₃CN / 0.1% H₃PO₄ gradient over 7 minutes then hold at 90% CH₃CN / 0.1% H₃PO₄ for 3 minutes; retention times of 6.04 minutes for the exo diastereomer and 5.66 minutes for the endo diastereomer. The mixture was washed with 1.5 M aqueous citric acid and recrystallized from MeOH to afford the title compound (71% yield) which was determined to be diastereomerically pure by HPLC analysis. ¹H NMR (DMSO-d₆, 300 MHz) δ 1.26-1.11(m), 1.29(m), 1.47(t), 1.59(m), 1.82(m), 3.62(d), 6.97(s), 7.27(s), 8.01(t); ¹³C NMR (DMSO-d₆, 100 MHz) δ 19.0, 20.7, 22.4, 26.1, 41.8, 171.1, 172.7; MS m/z 197 (M+H)⁺.

### Example 7

### 3-methylbicyclo[4.1.0]heptane-7-carboxylic acid

### Example 7A

### ethyl 3-methylbicyclo[4.1.0]heptane-7-carboxylate

The title compound was prepared as described in Example 5A substituting 4-methyl-1-cyclohexene, purchased from Aldrich, for cyclohexene.

### Example 7B

### 3-methylbicyclo[4.1.0]heptane-7-carboxylic acid

The title compound was prepared as described in Example 1B substituting the product from Example 7A for the product from Example 1A. MS (EI) m/z 154 (M)⁺.

### Example 8

### 3-methylbicyclo[4.1.0]heptane-7-carboxamide

The title compound was prepared as described in Example 3 substituting the product from Example 7B for the product from Example 1B. MS m/z 154 (M+H)⁺.

### Example 9

### N-(2-amino-2-oxoethyl)-3-methylbicylo[4.1.0]heptane-7-carboxamide

The title compound was prepared as described in Example 4 substituting the product from Example 7B for the product from Example 1B. MS m/z 211 (M+H)⁺.

### Example 10

### (exo) (1R,2R,4S,5S)-tricyclo[3.2.1.0^{2,4}]octane-3-carboxylic acid

### Example 10A

### ethyl (1R,2R,4S,5S)-tricyclo[3.2.1.0^{2,4}]octane-3-carboxylate

The title compound was prepared as described in Example 5A substituting norbornene, purchased from Aldrich, for cyclohexene.

### Example 10B

### (exo) (1R,2R,4S,5S)-tricyclo[3.2.1.0^{2,4}]octane-3-carboxylic acid

The title compound was prepared as described in Example 1B substituting the product from Example 10A for the product from Example 1A. The product was purified by chromatography (silica gel, 2.5% MeOH/CHCl₃) and recrystallized from EtOAc/hexame to afford the title compound, which was diastereomerically pure by HPLC analysis (conditions as described in Example 5C, retention time of 9.14 minutes). ¹H NMR (DMSO-d₆, 300 MHz) δ 0.63(d), 0.91 (dt), 1.20(d), 1.22-1.27(m), 1.40-1.45(m), 2.31(s), 11.93(s); ¹³C NMR (DMSO-d₆, 100 MHz) δ 15.5, 25.0, 28.1, 28.4, 35.2, 174.6; MS m/z 152 (M+H)⁺.

### Example 11

### (exo) (1R,2R,4S,5S) tricyclo[3.2.1.0^{2,4}]octane-3-carboxamide

The title compound was prepared as described in Example 3 substituting the product from Example 10B for the product from Example 1B. ¹H NMR (DMSO-d₆, 300 MHz) δ 0.64(d), 0.85 (dt), 1.05(d), 1.20-1.26(m), 1.39-1.44(m), 1.47(t), 2.27(s), 6.61(s), 7.32(s); ¹³C NMR (DMSO-d₆, 100 MHz) δ 16.4, 23.6, 28.4, 28.6, 35.1, 173.9; MS m/z 151(M+H)⁺.

### Example 12

### (exo) (1R,2R,4S,5S)-N-(2-amino-2-oxoethyl)tricyclo[3.2.1.0^{2,4}]octane-3-carboxamide

The title compound was prepared as described in Example 4 substituting the product from Example 10B for the product from Example 1B. ¹H NMR (DMSO-d₆, 300 MHz) δ 0.64(d), 0.85 (dt), 1.05(d), 1.20-1.26(m), 1.39-1.44(m), 1.47(t), 2.27(s), 6.61 (s), 7.32(s); ¹³C NMR (DMSO-d₆, 100 MHz) δ 16.4, 23.6, 28.4, 28.6, 35.1, 173.9; MS m/z 151(M+H)⁺. Stereochemistry was determined by several NMR techniques including by GDQCOSY, GHSQC, GHMBC and ROESY.

### Example 13

### 2,4-dimethylbicyclo[4.1.0]heptane-7-carboxylic acid

### Example 13A

### 3,5-dimethylcyclohexene

3,5-dimethylcyclohexanol (60 g, 468 mmol, purchased from Aldrich) was added to a solution of H₂O (240 mL) and concentrated sulfuric acid (120 mL) at such a rate that the temperature was maintained below 80 °C. The mixture was vigorously stirred at 100 °C for 12 hours and then cooled to ambient temperature and extracted with CH₂Cl₂ (360 mL). The organic extract was washed with 5% aqueous NaHCO₃ (2 x 200 mL), H₂O (200 mL), and concentrated by use of a rotary evaporator (bath temperature maintained below 30 °C) to provide a yellow oil (39.0 g). Distillation of the crude oil under reduced pressure afforded the title compound as a colorless oil (34.2 g, 66% yield).

### Example 13B

### ethyl 2,4-dimethylbicyclo[4.1.0]heptane-7-carboxylate

The title compound was prepared as described Example 1A substituting the product from Example 13A for (1S)-(+)-3-carene.

### Example 13C

### 2,4-dimethylbicyclo[4.1.0]heptane-7-carboxylic acid

The title compound was prepared as described Example 1B substituting the product from Example 13B for the product from Example 1A.

### Example 14

### 2,4-dimethylbicyclo[4.1.0]heptane-7-carboxamide

The title compound was prepared as described Example 3 substituting the product from Example 13C for the product from Example 1B. MS m/z 168 (M+H)⁺.

### Example 15

### N-(2-amino-2-oxoethyl)-2,4-dimethylbicylo[4.1.0]heptane-7-carboxamide

The title compound was prepared as described Example 4 substituting the product from Example 13C for the product from Example 1B. MS m/z 225 (M+H)⁺.

### Example 16

### (trans) 2,4-dimethylbicyclo[4.1.0]heptane-7-carboxylic acid

### Example 16A

### trans ethyl 2,4-dimethylbicylo[4.1.0]heptane-7-carboxylate

The title compound was prepared as described Example 5A substituting trans 3,5-dimethylcyclohexene, purchased from Wiley, for cyclohexene.

### Example 16B

### (trans) 2,4-dimethylbicyclo[4.1.0]heptane-7-carboxylic acid

The title compound was prepared as described Example 1B substituting the product from Example 16A for the product from Example 1A. ¹H NMR (DMSO-d₆, 300 MHz) δ 0.44(m), 0.77 (t), 0.82(d), 0.95(d), 1.05(d), 1.11-1.24(m), 1.28(t), 1.38(dd), 1.50(m), 1.65(m), 11.87(s).

### Example 17

### 2,4-dimethylbicyclo[4.1.0]heptane-7-carboxamide

The title compound can be prepared as described Example 3 substituting the product from Example 16B for the product from Example 1B.

### Example 18

### (1S,2S,4S,6R,7S)-N-(2-amino-2-oxoethyl)-2,4-dimethylbicyclo[4.1.0]heptane-7-carboxamide

The title compound was prepared as described Example 4 substituting the product from Example 16B for the product from Example 1B. The title compound was isolated as a single diastereomer by crystallization of the crude product from 1:1:3 EtOH/H₂O/MeOH. The stereochemistry of the title compound was determined by a variety of NMR spectroscopic techniques including GDQCOSY, GHSQC, GHMBC and ROESY. ¹H NMR (DMSO-d₆, 300 MHz) δ 0.43(q), 0.82(d), 0.95(m), 1.04(d), 1.07-1.22(m), 1.38(m), 1.46(t), 1.61(m), 1.84(d), 3.63(d), 6.97(s), 7.29(s), 7.99(t); ¹³C NMR (DMSO-d₆, 100 MHz) δ 21.9, 22.3, 23.6, 25.3, 26.0, 26.3, 30.2, 31.3, 40.9, 41.8, 171.1, 172.5. MS m/z 225 (M+H)⁺.

### Example 19

### (exo) (1aR,2S,2aS,5aR,6R,6aS)-decahydro-2,6-methanocyclopropa[f]indene-1-carboxylic acid

The title compound was prepared as described in Example 1A substituting (3aR,4R,7S,7aS)-2,3,3a,4,7,7a-hexahydro-1H-4,7-methanoindene for (1S)-(+)-3-carene. The crude product was recrystallized from EtOAc/hexane to provide the title compound (34% yield), which was diasteromerically pure by HPLC (conditions as described in Example 5C, retention time of 11.25 minutes). ¹H NMR (CDCl₃, 300 MHz) δ 0.95(d), 1.06(d), 1.52(m), 1.58(d), 1.61(m), 1.73(t), 1.83(m), 2.33(s), 2.39(m); ¹³C NMR (CDCl₃, 100 MHz) δ16.6, 24.2, 25.9, 30.1, 32.9, 39.7, 47.0, 181.2.

### Example 20

### (exo) (1aR,2R,2aS,5aR,65,6aS)-decahydro-2,6-methanocyclopropa[f]indene-1-carboxamide

The title compound was prepared as described in Example 3 substituting the product from Example 19 for the product from Example 1B (single diastereomer by HPLC: conditions as described in Example 5C, retention time of 9.89 minutes). ¹H NMR (DMSO-d₆, 300 MHz) δ 0.90(d), 1.03(d), 1.20(d), 1.46(m), 1.58(m), 1.67(t), 1.79(m), 2.21(s), 2.33(m), 6.66(s), 7.34(s); ¹³C NMR (DMSO-d₆, 100 MHz) δ 16.8, 20.4, 25.7, 29.8, 32.8, 39.0, 46.5, 173.9; MS m/z 192 (M+H)⁺.

### Example 21

### (exo) (1aR,2R,2aS,5aR,6S,6aS)-N-(2-amino-2-oxoethyl)decahydro-2,6-methanocyclopropa[f]indene-1-carboxamide

The title compound was prepared as described in Example 4 substituting the product from Example 19 for the product from Example 1B (single diastereomer by HPLC: conditions as described in Example 5C, retention time of 8.43 minutes). ¹H NMR (DMSO-d₆, 300 MHz) δ 0.90(d), 1.05(d), 1.22(d), 1.47(m), 1.58(m), 1.79(m), 1.83(t), 2.22(s), 2.35(m), 3.61(d), 6.97(s), 7.29(s), 8.05(t); ¹³C NMR (DMSO-d₆, 100 MHz) δ 17.0,20.5, 25.7,29.8, 32.8, 39.1,41.8,46.5, 171.0, 172.5; MS m/z 248 (M+H)⁺. Stereochemistry was determined by several NMR spectroscopic techniques including by GDQCOSY, GHSQC, GHMBC and ROESY.

### Example 22

### (1R,5S)-tricyclo[3.3.0.0^{2,4}]oct-2(4)-ene-3-carboxylic acid

### Example 22A

### (1R,5S)-bicyclo[3.2.0]hept-6-ene

1,3-Cycloheptadiene (purchased from Aldrich) was irradiated according to the procedure described by Arnold, A.; et. al. J. Amer. Chem. Soc. 1993, 115, 4271-4281 to provide the title compound.

### Example 22B

### ethyl (1R,5S)-tricyclo[3.3.0.0^{2,4}]oct-2(4)-ene-3-carboxylate

The title compound was prepared as described in Example 5A substituting the product from Example 22A for cyclohexene. The relative stereochemistry was determined by several NMR-spectroscopic techniques including GDQCOSY, GHSQC, GHMBC and ROESY.

### Example 22C

### (1R,5S)-tricyclo[3.3.0.0^{2,4}]oct-2(4)-ene-3-carboxylic acid

The title compoun was prepared as described in Example 1B substituting the product from Example 22B for the product from Example 1A. ¹H NMR (CDCl₃, 300 MHz) δ 1.43(m), 1.62(s), 1.68-1.74(m), 1.79(m), 2.06(m), 2.26(d), 5.95(broad s); ¹³C NMR(CDCl₃, 100 MHz) δ 25.0, 28.1, 30.1, 30.8, 42.6, 178.9; MS m/z 152 (M+H)⁺.

### Example 23

### (1R,5S)-tricyclo[3.3.0.0^{2,4}]oct-2(4)-ene-3-carboxamide

The title compound can be prepared as described in Example 3 substituting the product from Example 22C for the product from Example 1B.

### Example 24

### (1R,5S)-N-(2-amino-2-oxoethyl)tricyclo[3.3.0.0^{2,4}]oct-2(4)-ene-3-carboxamide

The title compound can be prepared as described in Example 4 substituting the product from Example 22C for the product from Example 1B. ¹H NMR (DMSO-d₆, 300 MHz) δ 1.41(m), 1.43(s), 1.70(q), 1.77(s), 1.79(m), 2.00(m), 2.21(d), 3.62(d), 6.94(s), 7.23(s), 8.13(t); ¹³C NMR (DMSO-d₆, 100 MHz) δ 24.6, 25.7, 30.0, 30.3, 41.6, 41.7, 170.5, 170.6; MS m/z 208 (M+H)⁺.

### Example 25

### (2S,5R)-2-isopropyl-5-methylbicyclo[4.1.0]heptane-7-carboxylic acid

### Example 25A

### ethyl (2S,5R)-2-isopropyl-5-methylbicyclo[4.1.0]heptane-7-carboxylate

The title compound was prepared as described in Example 5A substituting (+)-trans-p-menth-2-ene, purchased from Fluka, for cyclohexene.

### Example 25B

### (2S,5R)-2-isopropyl-5-methylbicylo[4.1.0]heptane-7-carboxylic acid

The title compound was prepared as described in Example 1B substituting the product from Example 25A for the product from Example 1A. MS m/z 214 (M+NH₄)⁺.

### Example 26

### (2S,5R)-2-isopropyl-5-methylbicylo[4.1.0]heptane-7-carboxamide

The title compound can be prepared as described in Example 3 substituting the product from Example 25B for the product from Example 1B.

### Example 27

### (2S,5R)-N-(2-amino-2-oxoethyl)-2-isopropyl-5-methylbicylo[4.1.0]heptane-7-carboxamide

The title compound was prepared as described in Example 4 substituting the product from Example 25B for the product from Example 1B. MS m/z 253 (M+NH₄)⁺.

### Example 28

### octahydro-1H-cyclopropa[a]pentalene-1-carboxylic acid

### Example 28A

### ethyl octahydro-1H-cyclopropa[a]pentalene-1-carboxylate

The title compound was prepared as described in Example 5A substituting 1,2,3,3a,4,6a-hexahydropentalene, purchased from Wiley, for cyclohexene.

### Example 28B

### octahydro-1H-cylopropa[a]pentalene-1-carboxylic acid

The title compound was prepared as described in Example 1B substituting the product from Example 28A for the product from Example 1A. The title compound was obtained in 17% yield by recrystallization from EtOAc/MeOH, as a single diastereomer by HPLC analysis (conditions as described in Example 5C, retention time of 10.29 minutes.) ¹H NMR (DMSO-d₆, 300 MHz) δ 1.04(t), 1.21-1.72(m), 1.90(d), 1.93(d), 2.10(t), 2.34(q), 3.58(d), 6.93(s), 7.25(s), 7.97(t); ¹³C NMR (DMSO-d₆, 100 MHz) δ 25.4, 26.5, 28.3, 31.8, 32.6, 32.9, 35.1, 40.8, 47.0, 174.8.

### Example 29

### octahydro-1H-cyclopropa[a]pentalene-1-carboxamide

The title compound can be prepared as described in Example 3 substituting the product from Example 28B for the product from Example 1B.

### Example 30

### N-(2-amino-2-oxoethyl)octahydro-1H-cyclopropa[a]pentalene-1-carboxamide

The title compound can be prepared as described in Example 4 substituting the product from Example 28B for the product from Example 1B. The title compound was obtained in 48% yield by recrystallization from EtOAC/MeOH as a single diastereomer by HPLC (conditions as described in Example 5C, retention time of 7.55 minutes). ¹H NMR (DMSO-d₆, 300 MHz) δ 1.28-1.74(m), 1.89(dd), 2.11(m), 2.34(m), 3.58(d), 6.93(s), 7.25(s), 7.97(t);¹³C NMR (DMSO-d₆, 100 MHz) δ 25.4, 26.2, 27.8, 31.8, 32.3, 32.6, 35.0, 40.9, 41.8, 46.9, 170.8, 171.7; MS m/z 223 (M+NH₄)⁺. The relative stereochemistry was determined by several NMR-spectroscopic techniques including GDQCOSY, GHSQC, GHMBC and ROESY.

### Example 31

### (endo) bicyclo[6.1.0]nonane-9-carboxylic acid

### Example 31A

### (endo) ethyl bicyclo[6.1.0]nonane-9-carboxylate

### (exo) ethyl bicylo[6.1.0]nonane-9-carboxylate

The title compounds were prepared as described in Example 5A substituting cyclooctene, purchased from Aldrich, for cyclohexene.

### Example 31B

### (endo) bicyclo[6.1.0]nonane-9-carboxylic acid

The product from Example 31A was processed according to the procedure described in Example 1B to afford a mixture of endo- and exo-bicyclo[6.1.0]nonane-9-carboxylic acids. A portion of this mixture of diastereomeric carboxylic acids (39.0 g, 0.232 mol) was dissolved in acetone (700 mL). (R)-(+)-α-Methylbenzylamine (29.5 mL, 0.232 mol, purchased from Aldrich) was added dropwise resulting in formation of a precipitate. The suspension was warmed to reflux and then cooled to room temperature and filtered. The mother liquors, enriched with the exo-isomer, were saved (see Example 32). The isolated solid (40.1 g) was recrystallized twice from acetone (15-20 mL/g) to afford the (R)-(+)-α-methylbenzylamine salt of the title compound (15.9 g) in 98% diastereomeric excess as determined by HPLC (conditions as described in Example 1A, retention time 11.46 minutes for the endo isomer and 11.24 minutes for the exo isomer). The (R)-(+)-α-methylbenzylamine salt of the title compound (15.9 g) was treated with 2 N aqueous HCl (27 mL, 1 mole equivalent) and extracted with ethyl acetate (50 mL). The organic extract was concentrated to afford the title compound (5.7 g). ¹H NMR (DMSO-d₆, 300 MHz) δ 1.12-1.22 (2H, m), 1.26-1.41 (4H, m), 1.49-1.61 (7H, m), 1.70-1.82 (2H, m), 11.68 (1H, br); ¹³C NMR (DMSO-d₆, 100 MHz) δ 20.2, 20.5, 23.6, 26.0, 28.8, 172.8; MS m/z 186 (M+NH₄)⁺. The stereochemistry of the title compound was determined by several NMR-spectroscopic techniques.

### Example 32

### (exo) bicyclo[6.1.0]nonane-9-carboxylic acid

The mother liquors of the (R)-(+)-α-methylbenzylamine salt from Example 31B, that were enriched with the exo-isomer (approximately 70% diastereomeric excess by HPLC) were concentrated to a solid (20 g). The solid was treated with 2N aqueous HCl (34 mL, 1 mole equivalent) and extracted with isopropyl acetate (100 mL, 5 mL/g). The organic extract was concentrated and the residue (11.9 g, 0.071 mol) was dissolved in acetone (230 mL, 20 mL/g). (S)-(-)-α-Methylbenzylamine (9.0 mL, 0.071 mol, purchased from Alrdich) was added dropwise resulting in formation of a precipitate. The suspension was warmed to reflux and then cooled to room temperature and filtered to afford the (S)-(+)-α-methylbenzylamine salt of the title compound (8.5 g, 97% diastereomeric excess as determined by HPLC). The salt was treated with 2N aqueous HCl (15 mL, 1 mole equivalent), extracted with isopropyl acetate (50 mL) and the organic extract was concentrated to afford the title compound (3.2 g). ¹H NMR (DMSO-d₆, 300 MHz) δ 0.98-1.10 (3H, m), 1.16-1.25 (2H, m), 1.28-1.41 (4H, m), 1.45-1.65 (4H, m), 1.96 (2H, dd), 11.81 (1H, br); ¹³C NMR (DMSO-d₆, 100 MHz) δ 25.0, 25.2, 26.0, 26.3, 28.7, 174.5; MS m/z 186 (M+NH₄)⁺. The stereochemistry of the title compound was determined by several NMR-spectroscopic techniques.

### Example 33

### (endo) bicyclo[6.1.0]nonane-9-carboxamide

The title compound can be prepared as described in Example 3 substituting the product from Example 31B for the product from Example 1B.

### Example 34

### (endo) N-(2-amino-2-oxoethyl)bicyclo[6.1.0]nonane-9-carboxamide

The title compound was prepared (66% yield) as described in Example 4 substituting the product from Example 31B for the product from Example 1B. ¹H NMR (DMSO-d₆, 300 MHz) δ 0.94-1.10 (2H, m), 1.21-1.40 (4H, m), 1.46-1.64 (7H, m), 1.75-1.92 (2H, m), 3.59 (2H, d), 6.94 (br, 1H, NH₂), 7.20 (1H, br, NH₂), 7.93 (1H, t, NH); ¹³C NMR (DMSO-d₆, 100 MHz) δ 20.7, 21.4, 22.7, 26.1, 29.1, 41.8, 170.3, 170.6; MS m/z 225 (M+H)⁺.

### Example 35

### (exo) bicyclo[6.1.0]nonane-9-carboxamide

The title compound can be prepared as described in Example 3 substituting the product from Example 32 for the product from Example 1B.

### Example 36

### (exo) N-(2-amino-2-oxoethyl)bicylo[6.1.0]nonane-9-carboxamide

The title compound was prepared (93% yield) as described in Example 4 substituting the product from Example 32 for the product from Example 1B. ¹H NMR (DMSO-d₆, 300 MHz) δ 0.97-1.17 (5H, m), 1.27-1.41 (4H, m), 1.46-1.67 (4H, m), 1.93 (2H, d), 3.61 (2H, d), 6.95&7.25 (2H, br, NH₂), 8.01 (1H, s, NH); ¹³C NMR (DMSO-d₆. 100 MHz) δ 24.6, 25.4, 26.0, 26.5, 28.9, 41.8, 170.4, 171.6; MS m/z 225 (M+H)⁺.

### Example 37

### 2,7,7-trimethyltricyclo[4.1.1.0^{2,4}]octane-3-carboxylic acid

### Example 37A

### ethyl 2,7,7-trimethyltricyclo[4.1.1.0^{2,4}]octane-3-carboxylate

The title compound was prepared as described in Example 1A substituting (1R)-(+)-α-pinene, purchased from Aldrich, for (1S)-(+)-3-carene.

### Example 37B

### 2,7,7-trimethyltricyclo[4.1.1.0^{2,4}]octane-3-carboxylic acid

The title compound was prepared as described in Example 1B substituting the product from Example 37A for the product from Example 1A. ¹H NMR (CDCl₃, 300 MHz) δ 1.02(s), 1.24(s), 1.29(s), 1.61(dd), 1.70(m), 1.76(m), 1.99(t), 2.05(d), 2.062.15(m); ¹³C NMR (CDCl₃, 100 MHz) δ 18.8, 20.8, 25.0, 26.2, 26.7, 26.7, 29.9, 32.9, 40.8, 40.8, 47.1, 178.7.

### Example 38

### 2,7,7-trimethyltricyclo[4.1.1.0^{2,4}]octane-3-carboxamide

The title compound was prepared as described in Example 3 substituting the product from Example 37B for the product from Example 1B. ¹H NMR (CDCl₃, 300 MHz) δ 1.00(d), 1.02(s), 1.18(s), 1.29(s), 1.58(dd), 1.70(m), 1.73(d),1.84(d), 1.95(t), 2.06-2.15(m), 5.57(br s); ¹³C NMR (CDCl₃, 100 MHz) δ 18.6, 20.8, 22.2, 26.2, 26.7, 26.8, 30.5, 31.7, 40.8, 41.0, 46.9, 174.4; MS m/z 154 (M+H)⁺.

### Example 39

### N-(2-amino-2-oxoethyl)-2,7,7-trimethyltricyclo[4.1.1.0^{2,4}]octane-3-carboxamide

The title compound was prepared as described in Example 3 substituting the product from Example 37B for the product from Example 1B. ¹H NMR (DMSO-d₆, 300 MHz) δ 0.99(s), 1.06(s), 1.10(d), 1.26(s), 1.37(dd), 1.62-1.66(m), 1.88(t), 1.98-1.06(m), 2.09(d), 3.64(t), 6.96(br s), 7.25(br s), 8.04(t); ¹³C NMR (DMSO-d₆, 100 MHz) δ 18.4, 20.7, 20.7, 25.8, 26.1, 26.6, 28.7, 30.8, 40.2, 40.4, 41.9, 46.6, 171.1, 171.2; MS m/z 251 (M+H)⁺.

### Example 40

### 1-methylbicylo[4.1.0]heptane-7-carboxylic acid

### Example 40A

### ethyl 1-methylbicyclo[4.1.0]heptane-7-carboxylate

The title compound was prepared as described in Example 1A substituting 1-methyl-1-cyclohexene, purchased from Aldrich, for (1S)-(+)-3-carene.

### Example 40B

### 1-methylbicyclo[4.1.0]heptane-7-carboxylic acid

The title compound was prepared as described in Example 1B substituting the product from Example 40A for the product from Example 1A. The title compound was isolated by crystallization from EtOAc/heptane (single isomer by HPLC: conditions described in Example 1A, retention time of 9.78 minutes). ¹H NMR (CDCl₃, 300 MHz) δ 1.17(m), 1.26(s), 1.28(m), 1.39(m), 1.50(d), 1.56-1.67(m), 1.84-1.99(m); ¹³C NMR (CDCl₃, 100 MHz) δ 20.8, 20.8, 21.1, 22.9, 29.0, 29.1, 30.2, 31.9, 178.7.

### Example 41

### 1-methylbicyclo[4.1.0]heptane-7-carboxamide

The title compound was prepared as described in Example 3 substituting the product from Example 40B for the product from Example 1B. ¹H NMR (CDCl₃, 300 MHz) δ 1.10-1.24(m), 1.24(s), 1.28(d), 1.35-1.43(m), 1.52-1.67(m), 1.78-1.84(m), 1.92-2.01(m); MS m/z 154 (M+H)⁺.

### Example 42

### N-(2-amino-2-oxoethyl)-1-methylbicylo[4.1.0]heptane-7-carboxamide

The title compound was prepared as described in Example 4 substituting the product from Example 40A for the product from Example 1B. ¹H NMR (CDCl₃, 300 MHz) δ 1.10-1.29(m), 1.17(s), 1.32(d), 1.34-1.42(m), 1.521.70(m), 1.77-1.83(m), 1.91-1.99 (m), 3.98(d), 5.53(broad s), 6.44(broad s); ¹³C NMR (CDCl₃, 100 MHz) δ 20.7, 21.0, 21.2, 22.9, 26.0, 26.8, 31.9, 32.3, 43.4, 171.6, 172.9; MS m/z 211 (M+H)⁺.

### Example 43

### (exo) bicyclo[5.1.0]octane-8-carboxamide

### Example 43A

### (exo) ethyl bicyclo[5.1.0]octane-8-carboxylate

The title compound was prepared as described in Example 1A substituting cycloheptene, purchased from Aldrich, for (1S)-(+)-3-carene.

### Example 43B

### (exo) bicyclo[5.1.0]octane-8-carboxamide

The title compound was prepared as described in Example 1B substituting the product from Example 43A for the product from Example 1A. The title compound was purified by crystallization from EtOAc and isolated as a single diastereomer (as determined by HPLC, conditions described in Example 1A, retention time of 9.70 minutes). ¹H NMR (CDCl₃, 300 MHz) δ 1.06-1.17(m), 1.19-1.27(m), 1.33-1.43(m) 1.59-1.71(m), 1.77-1.82(m), 2.15-2.21(m); ¹³C NMR (CDCl₃,100 MHz) δ 28.5, 28.8, 29.3, 29.5, 32.3, 180.4;MS m/z 154 (M)⁺.

### Example 43C

### (exo) bicyclo[5.1.0]octane-8-carboxamide

The title compound was prepared as described in Example 3 substituting the product from Example 43B for the product from Example 1B. ¹H NMR (DMSO-d₆, 300 MHz) δ 1.10-1.15(m), 1.17-1.38(m), 1.59-1.78(m), 2.02-2.12(m),6.62(br s), 7,35(br s); ¹³C NMR (DMSO-d₆, 100 MHz) δ 25.0, 28.6, 28.9, 30.3, 31.9, 173.8; MS m/z 153 (M)⁺.

### Example 44

### (exo) N-(2-amino-2-oxoethyl)bicyclo[5.1.0]octane-8-carboxamide

The title compound was prepared as described in Example 4 substituting the product from Example 43B for the product from Example 1B. ¹H NMR (DMSO-d₆, 300 MHz) δ 1.11-1.17(m), 1.18-1.38(m), 1.46(t) 1.59-1.75(m), 2.02-2.12(m), 3.62(d), 6.95(br s), 7.27(br s), 8.03(t); ¹³C NMR (DMSO-d₆, 100 MHz) δ 25.1, 28.6, 28.9, 30.3, 31.9, 41.8, 171.1, 172.0; MS m/z 211 (M+H)⁺.

### Example 45

### (exo) bicyclo[3.1.0]hexane-6-carboxylic acid

### Example 45A

### (exo) ethyl bicyclo[3.1.0]hexane-6-carboxylate

The title compound was prepared as described in Example 5A substituting cyclopentene, purchased from Aldrich, for cyclohexene.

### Example 45B

### (exo) bicyclo[3.1.0]hexane-6-carboxylic acid

The title compound was prepared as described in Example 1B substituting the product from Example 45A for the product from Example 1A. Crystallization from heptane afforded the title compound as a single diastereomer by HPLC (conditions described in Example 1A, retention time of 7.38 minutes). ¹H NMR CDCl₃, 300 MHz) δ 1.02-1.15(m), 1.39(t), 1.57-1.64(m), 1.71-1.94(m); ¹³C NMR (CDCl₃, 100 MHz) δ 20.0, 21.3, 27.2, 29.6, 180.5; MS m/z 144 (M+NH₄)⁺.

### Example 46 bicyclo[3.1.0]hexane-6-carboxamide

The title compound can be prepared as described in Example 3 substituting the product from Example 45B for the product from Example 1B.

### Example 47

### (exo) N-(2-amino-2-oxoethyl)bicyclo[3.1.0]hexane-6-carboxamide

The title compound can be prepared as described in Example 4 substituting the product from Example 45B for the product from Example 1B. ¹H NMR CDCl₃, 300 MHz) δ 0.98-1.11(m), 1.47(t), 1.52-1.58(m), 1.63-1.75(m), 3.62(d), 6.98(br s), 7.28(br s), 7.99(t);¹³C NMR (CDCl₃, 100 MHz) δ 20.3, 21.8, 26.0, 26.8, 41.7, 171.1, 172.1; MS m/z 183 (M+NH₄)⁺.

### Example 48

### 4,7,7-trimethyltricyclo[4.1.1.0^{2,4}]octane-3-carboxylic acid

### Example 48A

### ethyl 4,7,7-trimethyltricyclo[4.1.1.0^{2,4}]octane-3-carboxylate

The title compound was prepared as described in Example 1A substituting (1S)-(-)-α-pinene, purchased from Aldrich, for (1S)-(+)-3-carene.

### Example 48B

### 4,7,7-trimethyltricyclo[4.1.1.0^{2,4}]octane-3-carboxylic acid

The title compound was prepared as described in Example 1B substituting the product from Example 48A for the product from Example 1A. ¹H NMR (CDCl₃, 300 MHz) δ 1.02(s), 1.24(s), 1.29(s), 1.61(dd), 1.70(m), 1.76(m), 1.99(t), 2.05(d), 2.062.15(m).

### Example 49

### 4,7,7-trimethyltricyclo[4.1.1.0^{2,4}]octane-3-carboxamide

The title compound can be prepared as described in Example 3 substituting the product of Example 48B for the product from Example 1B.

### Example 50

### N-(2-amino-2-oxoethyl)-4,7,7-trimethyltricyclo[4.1.1.0^{2,4}]octane-3-carboxamide

The title compound can be prepared as described in Example 4 substituting the product from Example 48B for the product from Example 1B. ¹H NMR (DMSO-d₆, 300 MHz) δ 0.99(s), 1.06(s), 1.10(d), 1.26(d), 1.37(dd), 1.62-1.66(m), 1.88(t), 1.98-2.05(m), 2.09(d), 3.58-3.69(m), 6.96(br s), 7.25(br s), 8.04(t); ¹³C NMR (DMSO-d₆, 100 MHz) δ 18.4, 20.7, 20.7, 25.8, 26.1, 26.6, 28.7, 30.8, 40.2, 40.4, 41.9, 46.6, 171.1, 171.2; MS m/z 251 (M+H)⁺.

### Example 51

### 3-tert-butylbicyclo[4.1.0]heptane-7-carboxylic acid

### Example 51A

### 4-tert-butylcyclohexene

4-tert-butylcyclohexanol (20 g, 128 mmol, purchased from Aldrich) and triphenylphosphate (45.9 g, 141 mmol, purchased from Aldrich) were combined in 1-methylpyrrolidinone (120 mL) and heated at 190 °C for 72 hours and then at 200 °C for another 52 hours. The mixture was cooled to room temperature, diluted with H₂O (250 mL) and extracted with CH₂Cl₂ (100 mL). The organic extract was washed with 1N aqueous HCl (100 mL), H₂O (2 x 200 mL) and concentrated to give a dark brown oil (50.3 g). The crude product was distilled under reduced pressure to afford the title compound as a colorless oil (13.0 g, 74% yield).

### Example 51B

### ethyl 3-tert-butylbicyclo[4.1.0]heptane-7-carboxylate

The title compound was prepared as described in Example 1A by substituting the product from Example 51A for (1S)-(+)-3-carene. MS m/z 197 (M+H)⁺.

### Example 51C

### 3-tert-butylbicyclo[4.1.0]heptane-7-carboxylic acid

The title compound was prepared as described in Example 1B substituting the product from Example 51B for the product from Example 1A.

### Example 52

### 3-tert-butylbicyclo[4.1.0]heptane-7-carboxamide

The title compound was prepared as described in Example 3 substituting the product from Example 51C for the product from Example 1B. MS m/z 196 (M+H)⁺.

### Example 53

### N-(2-amino-2-oxoethyl)-3-tert-butylbicyclo[4.1.0]heptane-7-carboxamide

The title compound was prepared as described in Example 4 substituting the product from Example 51C for the product from Example 1B. MS m/z 253 (M+H)⁺.

### Example 54

### (1R,2R,4R,7R)-4,8,8-trimethyltricyclo[5.1.0.0^{2,4}]octane-3-carboxylic acid

### Example 54A

### ethyl (1R,2R,4R,7R)-4,8,8-trimethyltricyclo[5.1.0.0^{2,4}]octane-3-carboxylate

The title compound was prepared as described in Example 1A substituting (+)-2-carene, purchased from Aldrich, for (1S)-(+)-3-carene.

### Example 54B

### (1R,2R,4R,7R)-4,8,8-trimethyltricyclo[5.1.0.0^{2,4}]octane-3-carboxylic acid

The title compound was prepared as described in Example 1B substituting the product from Example 54A for the product from Example 1A (single isomer by HPLC analysis: conditions described in Example 1A, retention time of 12.1 minutes). ¹H NMR (DMSO-d₆, 300 MHz) δ 0.57(broad s), 0.92(s), 0.94-1.03(m), 1.00 (s), 1.05(s), 1.21(d), 1.54-1.59(m), 1.62-1.71(m); ¹³C NMR (DMSO-d₆, 100 MHz) δ 15.9, 16.3, 18.4, 18.5, 19.5, 21.2, 22.8, 24.7, 28.0, 31.4, 34.9, 174.2; MS m/z 195 (M+H)⁺.

### Example 55

### (1R,2R,4R,7R)-4,8,8-trimethyltricyclo[5.1.0.0^{2,4}]octane-3-carboxamide

The title compound was prepared as described in Example 3 substituting the product from Example 54B for the product from Example 1B. ¹H NMR (CDCl₃, 300 MHz) δ 0.57-0.65(m), 0.95(s), 1.02(s), 1.00-1.15(m), 1.13(s), 1.18(d), 1.33(s), 1.641.75(m), 5.54(broad s); ¹³C NMR (CDCl₃, 100 MHz) δ 15.9, 16.7, 18.3, 19.1, 19.8, 21.6, 23.6, 27.0, 28.2, 31.7, 35.1, 174.0; MS m/z 194 (M+H)⁺.

### Example 56

### (1R,2R,4R,7R)-N-(2-amino-2-oxoethyl)-4,8,8-trimethyltricyclo[5.1.0.0^{2,4}]octane-3-carboxamide

The title compound was prepared as described in Example 4 substituting the product from Example 54B for the product from Example 1B. ¹H NMR (CDCl₃, 300 MHz) δ 0.57-0.66(m), 0.96(s), 1.01(s), 1.04-1.17(m), 1.09(s), 1.23(d), 1.32(d), 1.62-1.79(m), 4.00(d), 5.62(br s), 6.58(br t); ¹³C NMR (CDCl₃, 100 MHz) δ 16.1, 16.8, 18.6, 19.3, 20.0, 21.7, 23.8, 27.1, 28.3, 31.6, 35.4, 43.3, 171.4, 172.2; MS m/z 251 (M+H)⁺.

### Example 57

### 1-methylbicyclo[3.1.0]hexane-6-carboxylic acid

### Example 57A

### ethyl 1-methylbicyclo[3.1.0]hexane-6-carboxylate

The title compound was prepared (90:10 ratio of exo/endo-diastereomers) as described in Example 1A substituting 1-methyl-1-cyclopentene, purchased from Aldrich, for (1S)-(+)-3-carene.

### Example 57B

### 1-methylbicyclo[3.1.0]hexane-6-carboxylic acid

The title compound was prepared as described in Example 1B substituting the product from Example 57A for the product from Example 1A. A ratio of 90:10 exo:endo-diastereomers was determined by HPLC analysis (conditions described in Example 1A, retention time 8.74 minutes for the exo isomer and 7.92 minutes for the endo isomers, respectively); ¹H NMR (CDCl₃, 300 MHz) δ 1.11-1.21(m), 1.29(s), 1.38(s), 1.51(d), 1.55-1.75(m), 1.76-1.84(m), 1.87-1.92(two d), 1.93-2.06(m); MS m/z 158 (M+NH₄)⁺.

### Example 58

### 1-methylbicyclo[3.1.0]hexane-6-carboxamide

The title compound was prepared as described in Example 3 substituting the product from Example 57B for the product from Example 1B. Crystallization from EtOAc/EtOH afforded the title compound as a single diastereomer by HPLC analysis (HPLC conditions described in Example 1A, retention time of 7.0 minutes). ¹H NMR (DMSO-d₆, 300 MHz) δ 1.05-1.18(m), 1.23(s), 1.41(d), 1.50(t), 1.54-1.77(m), 6.63(br s), 7.27(br s); ¹³C NMR (DMSO-d₆, 100 MHz) δ 14.8, 20.9, 26.2, 27.4, 30.1, 32.2, 35.1, 172.3; MS m/z 140 (M+H)⁺.

### Example 59

### N-(2-amino-2-oxoethyl)-1-methylbicyclo[3.1.0]hexane-6-carboxamide

The title compound was prepared as described in Example 4 substituting the product from Example 57B for the product from Example 1B. Crystallization from EtOAc/EtOH afforded the title compound as a single diastereomer by HPLC analysis (HPLC conditions described in Example 1A, retention time of 5.88 minutes). ¹H NMR (DMSO-d₆, 300 MHz) δ 1.05-1.17(m), 1.21(s), 1.32-1.78(m), 3.55(d), 3.60(d), 3.65(d), 3.69(d), 6.95(br s), 7.23(br s),7.93(t); ¹³C NMR (DMSO-d₆, 100 MHz) δ 14.8, 20.8, 26.4, 27.5, 30.3, 32.4, 35.1, 41.9, 170.5, 170.8; MS m/z 197 (M+H)⁺.

### Example 60

### 1,5-dimethylbicyclo[4.1.0]heptane-7-carboxylic acid

### Example 60A

### 1,3-dimethylcyclohexene

2,6-dimethylcyclohexanol (50.0 g, 390 mmol, purchased from Aldrich) was added to a solution of H₂O (200 mL) and concentrated sulfuric acid (120 mL) at such a rate that the temperature was maintained below 80 °C. The mixture was stirred at 100 °C for 30 hours then cooled to ambient temperature and extracted with CH₂Cl₂ (250 mL). The organic extract was washed with 5% aqueous NaHCO₃ (2 x 200 mL) and H₂O (200 mL), and then concentrated by use of a rotary evaporator (bath temperature less than 30 °C) to provide a brown oil (28.4 g). Distillation of the crude oil under reduced pressure afforded the title compound as a colorless oil (25 g, 58% yield).

### Example 60B

### ethyl 1,5-dimethylbicyclo[4.1.0]heptane-7-carboxylate

The title compound was prepared as described in Example 1A substituting the product from Example 60A for (1S)-(+)-3-carene.

### Example 60B

### 1,5-dimethylbicyclo[4.1.0]heptane-7-carboxylic acid

The title compound was prepared as described in Example 1B substituting the product from Example 60A for the product from Example 1A. MS m/z 168 (M)⁺.

### Example 61

### 1,5-dimethylbicyclo[4.1.0]heptane-7-carboxamide

The title compound was prepared as described in Example 3 substituting the product from Example 60B for the product from Example 1B. MS m/z 168 (M+H)⁺.

### Example 62

### N-(2-amino-2-oxoethyl)-1,5-dimethylbicyclo[4.1.0]heptane-7-carboxamide

The title compound was prepared as described in Example 4 substituting the product from Example 60B for the product from Example 1B. MS m/z 225 (M+H)⁺.

### Determination of anticonvulsant effect

The anticonvulsant effect of a representative number of compounds of the present invention were determined using the procedures described hereinafter.

Adult, male, CD-1 mice (22-25 grams) were obtained from Charles River Laboratories (Portage, MI) and housed at Abbott Laboratories (Abbott Park) under standard lighting conditions of 12 hours on/12 hours off, with lights on at 6 a.m. Food and water were provided ad libitum and mice weighed 25-35 grams at the time of testing.

Compounds were prepared for oral administration by suspending them in a vehicle of 100 µL Tween^{®} 80 per mL hydroxypropyl methylcellulose (2 mg/mL, Abbott Laboratories). Compound solutions were administered at a volume of 10 mL/kg, p.o.

### Maximal Electroshock Procedure:

The method used was similar to that of E.A. Swinyard, General principles: Experimental selection, quantification and evaluation of anti-convulsants, Anti-epileptic Drugs, Third Edition, R. Levey, et al., Editors. 1989, Raven Press Ltd: New York. Mice were pretreated orally with compounds of the present invention 30 minutes prior to electrical stimulation. Electrical stimulation consisted of pulsed electrical current (50 mA, 0.4 second duration, pulse width 0.5 msec, 60 pulses/sec) applied via corneal electrodes to induce seizure. The stimulation was delivered with an ECT Unit (Ugo Basile #7801). The electrodes of the unit were coated with electrocardiogram electrolyte (Signa Creme, Parker Labs #1708) to insure good contact with the corneas. Mice were observed post-stimulation for the onset of tonic seizures and death. Mice were considered to have had a tonic seizure only if there was a prolonged extension (>90° from plane of body) of the hind legs. Mice were assigned scores of either "positive" or "negative." A positive score indicated that the symptom was present; a negative that it was not. Those that did not seize were considered protected. A total of 20 mice were used in each group. The percent protection from tonic seizures was calculated by dividing the number of protected mice by the total number in the group. The ED₅₀ for the compounds were calculated using PROBIT analysis and represent the dose at which 50% of the mice were protected from tonic seizures. Valproate exhibited an ED₅₀ of 1.2 mmol/kg. Representative compounds of the present invention exhibited ED₅₀ₛ in the range of about 0.56 mmol/kg to about 0.05 mmol/kg.

### Subcutaneous pentylenetetrazole (PTZ) Seizure Procedure:

The method used was similar to that of E. A. Swinyard, General principles: Experimental selection, quantification and evaluation of anti-convulsants, Anti-epileptic Drugs, Third Edition, R. Levey, et al., Editors. 1989, Raven Press Ltd: New York. During the experiment the mice were housed individually in clear polycarbonate cages for observation. Mice, excluding control, were pretreated orally with a compound of the present invention 30 minutes prior to PTZ injection and were observed for 15 minutes following administration of PTZ. Seizures were induced by the subcutaneous injection of pentylenetetrazole (PTZ, 85 mg/kg) just below the nape of the neck. Time to clonic and tonic seizures was noted, and the number of mice that exhibited seizures was recorded. A total of 20 mice were used in each group. The ED₅₀for the compounds were calculated using linear regression and represent the dose at which 50% of the mice were protected from tonic seizures. Valproate exhibited an ED₅₀ of 1.8 mmol/kg. Representative compounds of the present invention exhibited ED₅₀ₛ in the range of about 0.79 mmol/kg to about 0.28 mmol/kg.

Compounds of the present invention can be used to treat seizures including, but not limited to, epilepsy as described by Schmidt, D., The clinical impact of new antiepileptic drugs after a decade of use in epilepsy, Epilepsy Res., 2002, 50(1-2), 21-32; Asconape, J. J., Some common issues in the use of antiepileptic drugs, Seminars in Neurology, 2002, 22(1), 27-39; and Wallace, S. J., Newer antiepileptic drugs: advantages and disadvantages, Brain & Development, 2001, 23, 277-283.

Compounds of the present invention can be used to treat bipolar disorder as described by Brambilla, P., Barale, F., Soares, J. C., Perspectives on the use of anticonvulsants in the treatment of bipolar disorder, International Journal of Neuropsychopharmacology, 2001, 4, 421-446; Angel, I. and Horovitz, T., Bipolar disorder and valproic acid, Current Opinion in Central & Peripheral Nervous System Investigational Drugs (1999), 1(4), 466-469; Muzina, D. J., El-Sayegh, S., Calabrese, J. R., Antiepileptic drugs in psychiatry-focus on randomized controlled trial, Epilepsy Research, 2002, 50 (1-2), 195-202; and Calabrese, J. R., Shelton, M. D., Rapport, D. J., Kimmel, S. E., Bipolar disorders and the effectiveness of novel anticonvulsants, J. Clin. Psychiatry, 2002, 63 (suppl 3), 5-9.

Compounds of the present invention can be used to treat psychiatric disorders including, but not limited to, anxiety and panic disorders, post-traumatic stress disorder, schizophrenia, episodic dyscontrol, substance-abuse-related disorders, impulse control disorders, general agitation associated with a variety of psychiatric disorders and dementias, and behavioral disorders associated with autism as described in Bialer, M., Johannessen, S. I., Kupferberg, H. J., Levy, R. H., Loiseau, P., Perucca, E., Progress report on new antiepileptic drugs: a summary of the sixth eilat conference (EILAT VI), Epilepsy Res. 2002, 51, 31-71; Fountain, N. B., Dreifuss, F. E., The future of valproate. In: Valproate.,Löscher W., Editor. 1999, Birkhauser Verlag, Boston; Fountain, N. B., Dreifuss, F. E., The future of valproate. In: Valproate., Löscher W., Editor. 1999, Birkhauser Verlag, Boston; and Balfour, J. A., Bryson, H. M. Valproic acid: A review of its pharmacology and therapeutic potential in indications other than epilepsy, CNS Drugs, 1994, 2 (2), 144-173.

Compounds of the present can be used to treat different types of migraine such as classical migraine and common migraine as described in Wheeler, S. D., Antiepileptic drugs therapy in migraine headache, Current Treatment Options in Neurology, 2002, 4, 383-394; and Krymchantowski, A. V., Bigal, M. E., Moreira, P. E., New and emerging prophylactic agents for migraine, CNS Drugs, 2002, 16 (9), 611-634.

Compounds of the present invention can be used to treat pain including, but not limited to, neuropathic pain including, but no limited to, diabetic neuropathy, cancer neuropathy, HIV pain, trigeminal neuralgia, post-herpetic neuralgia, traumatic neuralgia, phantom limb, severe refractory pain, and lancinating pain as described in Tremont-Lukats, I. W., Megeff, C., Backonja, M.-M., Anticonvulsants for neuropathich pain syndromes: mechanisms of action and place in therapy, Drugs, 60 (5), 1029-1052; Jensen, T. S., Anticonvulsants in neuropathic pain: rationale and clinical evidence, Eur. J. Pain, 2002, 6 (suppl A), 61-68; and Balfour, J. A., Bryson, H. M. Valproic acid: A review of its pharmacology and therapeutic potential in indications other than epilepsy, CNS Drugs, 1994, 2 (2), 144-173; Hardy, J. R., Rees, E. A. J., Gwilliam, B., Ling, J., Broadley, K., A'Hern, R., J. of Pain and Symptom Management, 2001, 21 (3), 204209.

Compounds of the present invention can be used to provide neuroprotection as described in Pitkanen, A., Efficacy of current antiepileptics to prevent neurodegeneration in epilepsy models, Epilepsy Research, 2002, 50, 141-160.

Compounds of the present invention can be used to treat movement disorders including, but not limited to, restless leg syndrome, periodic limb movements of sleep, essential tremor, acquired nystagmus, post-anoxic myoclonus, spinal myoclonus, spasticity, chorea, and dystonia as described in Magnus, L., Nonepileptic uses of gabapentin, Epilepsia, 1999, 40 (suppl 6), S66-S72; Fountain, N. B., Dreifuss, F. E., The future of valproate. In: Valproate., Löscher W., Editor. 1999, Birkhauser Verlag, Boston; Cutter, N., Scott, D. D., Johnson, J. C., Whiteneck, G., Gabapentin effect on spacticity in multiple sclerosis, 2000, 81, 164-169.

The present invention also provides pharmaceutical compositions that comprise compounds of the present invention. The pharmaceutical compositions comprise compounds of the present invention formulated together with one or more non-toxic pharmaceutically acceptable carriers.

The pharmaceutical compositions of this invention can be administered to humans and other mammals orally, rectally, parenterally , intracisternally, intravaginally, topically (as by powders, ointments or drops), bucally or as an oral or nasal spray. The term "parenterally," as used herein, refers to modes of administration which include intravenous, intramuscular, intraperitoneal, intrasternal, subcutaneous and intraarticular injection and infusion.

The term "pharmaceutically acceptable carrier," as used herein, means a non-toxic, inert solid, semi-solid or liquid filler, diluent, encapsulating material or formulation auxiliary of any type. Some examples of materials which can serve as pharmaceutically acceptable carriers are sugars such as, but not limited to, lactose, glucose and sucrose; starches such as, but not limited to, corn starch and potato starch; cellulose and its derivatives such as, but not limited to, sodium carboxymethyl cellulose, ethyl cellulose and cellulose acetate; powdered tragacanth; malt; gelatin; talc; excipients such as, but not limited to, cocoa butter and suppository waxes; oils such as, but not limited to, peanut oil, cottonseed oil, safflower oil, sesame oil, olive oil, corn oil and soybean oil; glycols; such as propylene glycol; esters such as, but not limited to, ethyl oleate and ethyl laurate; agar; buffering agents such as, but not limited to, magnesium hydroxide and aluminum hydroxide; alginic acid; pyrogen-free water; isotonic saline; Ringer's solution; ethyl alcohol, and phosphate buffer solutions, as well as other non-toxic compatible lubricants such as, but not limited to, sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), vegetable oils (such as olive oil), injectable organic esters (such as ethyl oleate) and suitable mixtures thereof. Proper fluidity can be maintained, for example, by the use of coating materials such as lecithin, by the maintenance of the required particle size in the case of dispersions and by the use of surfactants.

These compositions may also contain adjuvants such as preservatives, wetting agents, emulsifying agents and dispersing agents. Prevention of the action of microorganisms can be ensured by the inclusion of various antibacterial and antifungal agents, for example, paraben, chlorobutanol, phenol sorbic acid and the like. It may also be desirable to include isotonic agents such as sugars, sodium chloride and the like. Prolonged absorption of the injectable pharmaceutical form can be brought about by the inclusion of agents which delay absorption such as aluminum monostearate and gelatin.

In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, the active compound may be mixed with at least one inert, pharmaceutically acceptable carrier or excipient, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such carriers as lactose or milk sugar as well as high molecular weight polyethylene glycols and the like.

The solid dosage forms of tablets, dragees, capsules, pills and granules can be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned carriers.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, tragacanth and mixtures thereof.

Compositions for rectal or vaginal administration are preferably suppositories which can be prepared by mixing the compounds of this invention with suitable non-irritating carriers or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients and the like. The preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 et seq.

Dosage forms for topical administration of a compound of this invention include powders, sprays, ointments and inhalants. The active compound may be mixed under sterile conditions with a pharmaceutically acceptable carrier and any needed preservatives, buffers or propellants which may be required. Opthalmic formulations, eye ointments, powders and solutions are also contemplated as being within the scope of this invention.

Actual dosage levels of active ingredients in the pharmaceutical compositions of this invention can be varied so as to obtain an amount of the active compound(s) which is effective to achieve the desired therapeutic response for a particular patient, compositions and mode of administration. The selected dosage level will depend upon the activity of the particular compound, the route of administration, the severity of the condition being treated and the condition and prior medical history of the patient being treated.

When used in the above or other treatments, a therapeutically effective amount of one of the compounds of the present invention can be employed in pure form or, where such forms exist, in pharmaceutically acceptable salt, ester or prodrug form. The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The term "pharmaceutically acceptable prodrug" or "prodrug,"as used herein, represents those prodrugs of the compounds of the present invention which are, within the scope of sound medical judgement, suitable for use in contact with the tissues of humans and lower animals without undue toxicity, irritation, allergic response, and the like. Prodrugs of the present invention may be rapidly transformed in vivo to compounds of formula (I), for example, by hydrolysis in blood.

The present invention contemplates compounds of formula (I) formed by synthetic means or formed by in vivo biotransformation.

The compounds of the invention can exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

The total daily dose of the compounds of this invention administered to a human or lower animal may range from about 0.003 to about 90 mg/kg/day. For purposes of oral administration, more preferable doses can be in the range of from about 0.01 to about 30 mg/kg/day. If desired, the effective daily dose can be divided into multiple doses for purposes of administration; consequently, single dose compositions may contain such amounts or submultiples thereof to make up the daily dose.

## Claims

1. Use of a therapeutically effective amount of a compound of formula (I) 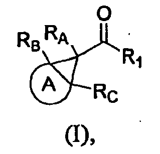 wherein
A is cycloalkyl or bicycloalkyl;
R_{A}, R_{B}, and R_{C} are independently hydrogen or alkyl;
R₁ is OR₂ or NR₃R₄;
R₂ is hydrogen or alkyl;
R₃ and R₄ are independently hydrogen, alkenyl, alkyl, alkynyl, alkoxycarbonylalkyl, aryl, arylalkyl, carboxyalkyl, cycloalkyl, cycloalkylalkyl, heterocycle, heterocyclealkyl, hydroxyalkyl, (NR₅R₆)alkyl, (NR₅R₆)carbonylalkyl, or 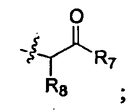 or
R₃ and R₄ taken together with the nitrogen atom to which they are attached form a heterocycle wherein the heterocycle is azepanyl, azetidinyl, aziridinyl, morpholinyl, piperazinyl, piperidinyl, pyrrolidinyl, or thiomorpholinyl;
R₅ and R₆ are independently hydrogen, alkenyl, alkyl, alkynyl, alkoxycarbonylalkyl, aryl, arylalkyl, cycloalkyl, cycloalkylalkyl, heterocycle, heterocyclealkyl, or hydroxyalkyl;
R₇ is alkoxy, alkyl, hydroxy, or -NR₅R₆;
R₈ is alkenyl, alkoxyalkyl, alkoxycarbonylalkyl, alkylthioalkyl, alkynyl, aryl, arylalkyl, carboxyalkyl, cycloalkyl, cycloalkylalkyl, heterocycle, heterocyclealkyl, hydroxyalkyl, mercaptoalkyl, (NR₅R₆)alkyl, (NR₅R₆)carbonylalkyl, or -(CH₂)ₙNHC(=NH)NH₂; and
n is an integer from 1 to 6;
provided that the compound of formula (I) is other than bicyclo[4.1.0]heptane-7-carboxylic acid,
for the manufacture of a medicament for treating migraine, epilepsy or bipolar disorder in a mammal.

2. The use according to claim 1 wherein
A is cycloalkyl; and
R₁ is OR₂.

3. The use according to claim 1 wherein
A is cycloalkyl wherein the cycloalkyl is optionally substituted with 1 or 2 alkyl groups ; and
R₁ is OR₂.

4. The use according to claim 3 wherein the compound of formula (I) is
3-methylbicyclo[4.1.0]heptane-7-carboxylic acid;
(exo) (1R,2R,4S,5S)-tricyclo[3.2.1.0^{2,4}]octane-3-carboxylic acid;
2,4-dimethylbicyclo[4.1.0]heptane-7-carboxylic acid;
(trans) 2,4-dimethylbicyclo[4.1.0]heptane-7-carboxylic acid;
(2S,5R)-2-isopropyl-5-methylbicyclo[4.1.0]heptane-7-carboxylic acid;
(endo) bicyclo[6.1.0]nonane-9-carboxylic acid;
(exo) bicyclo[6.1.0]nonane-9-carboxylic acid;
2,7,7-trimethyltricyclo[4.1.1.0^{2,4}]octane-3-carboxylic acid;
1-methylbicyclo[4.1.0]heptane-7-carboxylic acid;
(exo) bicyclo[3.1.0]hexane-6-carboxylic acid;
4,7,7-trimethyltricyclo[4.1.1.0^{2,4}]octane-3-carboxylic acid;
3-tert-butylbicyclo[4.1.0]heptane-7-carboxylic acid;
1-methylbicyclo[3.1.0]hexane-6-carboxylic acid; or
1,5-dimethylbicyclo[4.1.0]heptane-7-carboxylic acid.

5. The use according to claim 1 wherein
A is bicycloalkyl; and
R₁ is OR₂.

6. The use according to claim 1 wherein
A is bicycloalkyl wherein the bicycloalkyl is optionally substituted with 1 or 2 alkyl groups; and
R₁ is OR₂.

7. The use according to claim 6 wherein the compound of formula (I) is
(1S,3S,5S,7R)-3,8,8-trimethyltricyclo[5.1.0.0^{3,5}]octane-4-carboxylic acid;
(1S,3S,4R,7R)-3,8,8-trimethyltricyclo[5.1.0.0^{3,5}]octane-4-carboxylic acid;
(exo) (1aR,2R,2aS,5aR,6S,6aS)-decahydro-2,6-methanocyclopropa[f]indene-1-carboxylic acid;
(1R,5S)-tricyclo[3.3.0.0^{2,4}]oct-2(4)-ene-3-carboxylic acid;
octahydro-1H-cyclopropa[a]pentalene-1-carboxylic acid; or
(1R,2R,4R,7R)-4,8,8-trimethyltricyclo[5.1.0.0^{2,4}]octane-3-carboxylic acid.

8. The use according to claim 1 wherein
A is cycloalkyl; and
R₁ is NR₃R₄.

9. The use according to claim 1 wherein
A is cycloalkyl wherein the cycloalkyl is optionally substituted with 1 or 2 alkyl groups ;
R₁ is NR₃R₄;
R₃ is hydrogen;
R₄ is hydrogen or (NR₅R₆)carbonylalkyl; and
R₅ and R₆ are hydrogen.

10. The use according to claim 9 wherein the compound of formula (I) is
(exo) (1R,6S)-bicyclo[4.1.0]heptane-7-carboxamide;
(exo) (1R,6S)-N-(2-amino-2-oxoethyl)bicyclo[4.1.0]heptane-7-carboxamide;
3-methylbicyclo[4.1.0]heptane-7-carboxamide;
N-(2-amino-2-oxoethyl)-3-methylbicyclo[4.1.0]heptane-7-carboxamide;
(exo) (1R,2R,4S,5S)-tricyclo[3.2.1.0^{2,4}]octane-3-carboxamide;
(exo) (1R,2R,4S,5S)-N-(2-amino-2-oxoethyl)tricyclo[3.2.1.0^{2,4}]octane-3-carboxamide;
2,4-dimethylbicyclo[4.1.0]heptane-7-carboxamide;
N-(2-amino-2-oxoethyl)-2,4-dimethylbicyclo[4.1.0]heptane-7-carboxamide;
2,4-dimethylbicyclo[4.1.0]heptane-7-carboxamide;
(1S,2S,4S,6R,7S)-N-(2-amino-2-oxoethyl)-2,4-dimethylbicyclo[4.1.0]heptane-7-carboxamide;
(2S,5R)-2-isopropyl-5-methylbicyclo[4.1.0]heptane-7-carboxamide;
(2S,5R)-N-(2-amino-2-oxoethyl)-2-isopropyl-5-methylbicyclo[4.1.0]heptane-7-carboxamide;
(endo) bicyclo[6.1.0]nonane-9-carboxamide;
(endo) N-(2-amino-2-oxoethyl)bicyclo[6.1.0]nonane-9-carboxamide;
(exo) bicyclo[6.1.0]nonane-9-carboxamide;
(exo) N-(2-amino-2-oxoethyl)bicyclo[6.1.0]nonane-9-carboxamide;
2,7,7-trimethyltricyclo[4.1.1.0^{2,4}]octane-3-carboxamide;
N-(2-amino-2-oxoethyl)-2,7,7-trimethyltricyclo[4.1.1.0^{2,4}]octane-3-carboxamide;
1-methylbicyclo[4.1.0]heptane-7-carboxamide;
N-(2-amino-2-oxoethyl)-1-methylbicyclo[4.1.0]heptane-7-carboxamide;
(exo) bicyclo[5.1.0]octane-8-carboxamide;
(exo) N-(2-amino-2-oxoethyl)bicyclo[5.1.0]octane-8-carboxamide;
bicyclo[3.1.0]hexane-6-carboxamide;
(exo) N-(2-amino-2-oxoethyl)bicyclo[3.1.0]hexane-6-carboxamide;
4,7,7-trimethyltricyclo[4.1.1.0^{2,4}]octane-3-carboxamide;
N-(2-amino-2-oxoethyl)-4,7,7-trimethyltricyclo[4.1.1.0^{2,4}]octane-3-carboxamide;
3-tert-butylbicyclo[4.1.0]heptane-7-carboxamide;
N-(2-amino-2-oxoethyl)-3-tert-butylbicyclo[4.1.0]heptane-7-carboxamide;
1-methylbicyclo[3.1.0]hexane-6-carboxamide;
N-(2-amino-2-oxoethyl)-1-methylbicyclo[3.1.0]hexane-6-carboxamide;
1,5-dimethylbicyclo[4.1.0]heptane-7-carboxamide; or
N-(2-amino-2-oxoethyl)-1,5-dimethylbicyclo[4.1.0]heptane-7-carboxamide.

11. The use according to claim 1 wherein
A is bicycloalkyl; and
R₁ is NR₃R₄.

12. The use according to claim 1 wherein
A is bicycloalkyl wherein the bicycloalkyl is optionally substituted with 1 or 2 alkyl groups;
R₁ is NR₃R₄;
R₃ is hydrogen;
R₄ is hydrogen or (NR₅R₆)carbonylalkyl; and
R₅ and R₆ are hydrogen.

13. The use according to claim 12 wherein the compound of formula (I) is
(1S,3S,4S,7R)-3,8,9-trimethyltricyclo[5.1.0.0^{3,5}]octane-4-carboxamide;
(1S,3S,4S,7R)-N-(2-amino-2-oxoethyl)-3,8,8-trimethyltricyclo[5.1.0.0^{3,5}]octane-4-carboxamide;
(exo) (1aR,2R,2aS,5aR,6S,6aS)-decahydro-2,6-methanocyclopropa[f]indene-1-carboxamide;
(exo) (1aR,2R,2aS,5aR,6S,6aS)-N-(2-amino-2-oxoethyl)decahydro-2,6-methanocyclopropa[f]indene-1-carboxamide;
(1R,5S)-tricyclo[3.3.0.0^{2,4}]oct-2(4)-ene-3-carboxamide;
(1R,5S)-N-(2-amino-2-oxoethyl)tricyclo[3.3.0.0^{2,4}]oct-2(4)-ene-3-carboxamide;
octahydro-1H-cyclopropa[a]pentalene-1-carboxamide;
N-(2-amino-2-oxoethyl)octahydro-1H-cyclopropa[a]pentalene-1-carboxamide;
(1R,2R,4R,7R)-4,8,8-trimethyltricyclo[5.1.0.0^{2,4}]octane-3-carboxamide; or
(1R,2R,4R,7R)-N-(2-amino-2-oxoethyl)-4,8,8-trimethyltricyclo[5.1.0.0^{2,4}]octane-3-carboxamide.

14. Use of a therapeutically effective amount of a compound of formula (I), as defined in claim 1, for the manufacture of a medicament for treating a psychiatric disorder in a mammal.

15. Use of a therapeutically effective amount of a compound of formula (I), as defined in claim 1, forthe manufacture of a medicament for treating pain in a mammal.

16. Use of a therapeutically effective amount of a compound of formula (I), as defined in claim 1, for the manufacture of a medicament for treating a movement disorder in a mammal.

17. Use of a therapeutically effective amount of a compound of formula (I), as defined in claim 1, for the manufacture of a medicament for providing neuroprotection in a mammal.

18. A compound of formula (II) 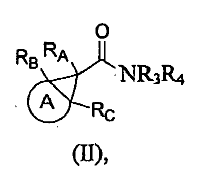 wherein
A is cycloalkyl wherein the cycloalkyl is optionally substituted with 1 or 2 alkyl groups;
R_{A}, R_{B}, and R_{C} are independently hydrogen or alkyl;
R₃ is hydrogen;
R₄ is (NR₅R₆)carbonylalkyl; and
R₅ and R₆ are hydrogen.

19. The compound according to claim 18 wherein the compound of formula (II) is
(exo) (1R,6S)-N-(2-amino-2-oxoethyl)bicyclo[4.1.0]heptane-7-carboxamide;
N-(2-amino-2-oxoethyl)-3-methylbicyclo[4.1.0]heptane-7-carboxamide;
(exo) (1R,2R,4S,5S)-N-(2-amino-2-oxoethyl)tricyclo[3.2.1.0^{2,4}]octane-3-carboxamide;
N-(2-amino-2-oxoethyl)-2,4-dimethylbicyclo[4.1.0]heptane-7-carboxamide;
(1S,2S,4S,6R,7S)-N-(2-amino-2-oxoethyl)-2,4-dimethylbicyclo[4.1.0]heptane-7-carboxamide;
(2S,5R)-N-(2-amino-2-oxoethyl)-2-isopropyl-5-methylbicyclo[4.1.0]heptane-7-carboxamide;
(endo) N-(2-amino-2-oxoethyl)bicyclo[6.1.0]nonane-9-carboxamide;
(exo) N-(2-amino-2-oxoethyl)bicyclo[6.1.0]nonane-9-carboxamide;
N-(2-amino-2-oxoethyl)-2,7,7-trimethyltricyclo[4.1.1.0^{2,4}]octane-3-carboxamide;
N-(2-amino-2-oxoethyl)-1-methylbicyclo[4.1.0]heptane-7-carboxamide;
(exo) N-(2-amino-2-oxoethyl)bicyclo[5.1.0]octane-8-carboxamide;
(exo) N-(2-amino-2-oxoethyl)bicyclo[3.1.0]hexane-6-carboxamide;
N-(2-amino-2-oxoethyl)-4,7,7-trimethyltricyclo[4.1.1.0^{2,4}]octane-3-carboxamide;
N-(2-amino-2-oxoethyl)-3-tert-butylbicyclo[4.1.0]heptane-7-carboxamide;
N-(2-amino-2-oxoethyl)-1-methylbicyclo[3.1.0]hexane-6-carboxamide; or
N-(2-amino-2-oxoethyl)-1,5-dimethylbicyclo[4.1.0]heptane-7-carboxamide.

20. A compound of formula (II) 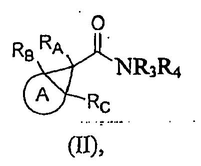 wherein
A is bicycloalkyl wherein the bicycloalkyl is optionally substituted with 1 or 2 alkyl groups; R_{A}, R_{B}, and R_{C} are independently hydrogen or alkyl;
R₃ is hydrogen;
R₄ is (NR₅R₆)carbonylalkyl; and
R₅ and R₆ are hydrogen.

21. The compound according to claim 20 wherein the compound of formula (II) is
(1S,3S,4S,7R)-N-(2-amino-2-oxoethyl)-3,8,8-trimethyltricyclo[5.1.0.0^{3,5}]octane-4-carboxamide;
(exo) (1aR,2R,2aS,5aR,6S,6aS)-N-(2-amino-2-oxoethyl)decahydro-2,6-methanocyclopropa[f]indene-1-carboxamide;
(1R,5S)-N-(2-amino-2-oxoethyl)tricyclo[3.3.0.0^{2,4}]oct-2(4)-ene-3-carboxamide;
N-(2-amino-2-oxoethyl)octahydro-1H-cyclopropa[a]pentalene-1-carboxamide; or
(1R,2R,4R,7R)-N-(2-amino-2-oxoethyl)-4,4,8-trimethyltricyclo[5.1.0.0^{2,4}]octane-3-carboxamide.

22. Use of a therapeutically effective amount of a compound of formula (1), as defined in claim 1, for the manufacture of a medicament for treating neuropathic and inflammatory pain in a mammal.

## Patentansprüche

1. Verwendung einer therapeutisch wirksamen Menge einer Verbindung mit der Formel (I) 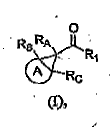 worin
A Cycloalkyl oder Bicycloalkyl ist;
R_{A}, R_{B} und R_{C} unabhängig Wasserstoff oder Alkyl sind;
R₁ OR₂ oder NR₃R₄ ist;
R₂ Wasserstoff oder Alkyl ist;
R₃ und R₄ unabhängig Wasserstoff, Alkenyl, Alkyl, Alkinyl, Alkoxycarbonylalkyl, Aryl, Arylalkyl, Carboxyalkyl, Cycloalkyl, Cycloalkylalkyl, Heterocyclus, Heterocyclusalkyl, Hydroxyalkyl, (NR₅R₆)Alkyl, (NR₅R₆)Carbonylalkyl; oder 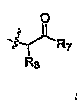 sind; oder
R₃ und R₄ zusammengenommen mit dem Stickstoffatom, an welches sie gebunden sind, einen Heterocyclus bilden, worin der Heterocyclus Azepanyl, Azetidinyl, Aziridinyl, Morpholinyl, Piperazinyl, Piperidinyl, Pyrrolidinyl oder Thiomorpholinyl ist;
R₅ und R₆ sind unabhängig Wasserstoff, Alkenyl, Alkyl, Alkinyl, Alkoxycarbonylalkyl, Aryl, Arylalkyl, Cycloalkyl, Cycloalkylalkyl, Heterocyclus, Heterocyclusalkyl oder Hydroxyalkyl;
R₇ ist Alkoxy, Alkyl, Hydroxy oder -NR₅R₆;
R₈ ist Alkenyl, Alkoxyalkyl, Alkoxycarbonylalkyl, Alkylthioalkyl, Alkinyl, Aryl, Arylalkyl, Carboxyalkyl, Cycloalkyl, Cycloalkylalkyl, Heterocyclus, Heterocyclusalkyl, Hydroxyalkyl, Mercaptoalkyl, (NR₅R₆)Alkyl, (NR₅R₆)Carbonylalkyl oder -(CH₂)ₙNHC(=NH)NH₂; und
n ist eine ganze Zahl von 1 bis 6;
vorausgesetzt, dass die Verbindung mit der Formel (I) nicht Bicyclo[4,1.0]heptan-7-carbonsäure ist,
für die Herstellung eines Medikaments für die Behandlung von Migräne, Epilepsie oder bipolarer Störung bei einem Säugetier.

2. Verwendung gemäß Anspruch 1, worin
A Cycloalkyl ist und
R₁ OR₂ ist.

3. Verwendung gemäß Anspruch 1, worin
A Cycloalkyl ist, worin das Cycloalkyl wahlweise substituiert ist mit 1 oder 2 Alkylgruppen,
und
R₁ OR₂ ist.

4. Verwendung gemäß Anspruch 3, worin die Verbindung mit der Formel (I)
3-Methylbicyclo[4.1.0]heptan-7-carbonsäure;
(exo) (1R,2R,4S,5S)-Tricyclo[3.2.1.0^{2,4}]octan-3-carbonsäure;
2,4-Dimethylbicyclo[4.1.0]heptan-7-carbonsäure;
(trans) 2,4-Dimethylbicyclo[4.1.0]heptan-7-carbonsäure;
(2S,5R)-2-Isopropyl-5-methylbicyclo[4.1.0]heptan-7-carbonsäure;
(endo) Bicyclo[6.1.0]nonan-9-carbonsäure;
(exo) Bicyclo[6.1.0]nonan-9-carbonsäure;
2,7,7-Trimethyltricyclo[4.1.1.0^{2,4}]octan-3-carbonsäure;
1-Methylbicyclo[4.1.0]heptan-7-carbonsäure;
(exo) Bicyclo[3.1.0]hexan-6-carbonsäure;
4,7,7-Trimethyltricyclo[4.1.1.0^{2,4}]octan-3-carbonsäure;
3-tert-Butylbicyclo[4.1.0]heptan-7-carbonsäure;
1-Methylbicyclo[3.1.0]hexan-6-carbonsäure oder
1,5-Dimethylbicyclo[4.1.0]heptan-7-carbonsäure ist.

5. Verwendung gemäß Anspruch 1, worin
A Bicycloalkyl ist und
R₁ OR₂ ist.

6. Verwendung gemäß Anspruch 1, worin
A Bicycloalkyl ist, worin das Bicycloalkyl wahlweise substituiert ist mit 1 oder 2 Alkylgruppen, und
R₁ OR₂ ist.

7. Verwendung gemäß Anspruch 6, worin die Verbindung mit der Formel (I)
(1S,3S,5S,7R)-3,8,8-Trimethyltricyclo[5.1.0.0^{3,5}]octan-4-carbonsäure;
(1S,3S,4R,7R)-3,8,8-Trimethyltricyclo[5.1.0.0^{3,5}]octan-4-carbonsäure;
(exo)(1aR,2R,2aS,5aR,6S,6aS)-Decahydro-2,6-methanocyclopropa[f]inden-1-carbonsäure;
(1R,5S)-Tricyclo[3.3.0.0^{2,4}]oct-2(4)-en-3-carbonsäure;
Octahydro-1H-cyclopropa[a]pentalen-1-carbonsäure oder
(1R,2R,4R,7R)-4,8,8-Trimethyltricyclo[5.1.0.0^{2,4}]octan-3-carbonsäure ist.

8. Verwendung gemäß Anspruch 1, worin
A Cycloalkyl ist und
R₁ NR₃R₄ ist.

9. Verwendung gemäß Anspruch 1, worin
A Cycloalkyl ist, worin das Cycloalkyl wahlweise substituiert ist mit 1 oder 2 Alkylgruppen;
R₁ NR₃R₄ ist;
R₃ Wasserstoff ist;
R₄ Wasserstoff oder (NR₅R₆)Carbonylalkyl ist und
R₅ und P₆ Wasserstoff sind.

10. Verwendung gemäß Anspruch 9, worin die Verbindung mit der Formel (I)
(exo) (1R,6S)-Bicyclo[4,1.0]heptan-7-carboxamid;
(exo) (1R,6S)-N-(2-Amino-2-oxoethyl)bicyclo[4.1.0]heptan-7-carboxamid;
3-Methylbicyclo[4.1.0]heptan-7-carboxamid;
N-(2-Amino-2-oxoethyl)-3-methylbicyclo[4.1.0]heptan-7-carboxamid;
(exo) (1R,2R,4S,5S)-Tricyclo[3.2.1.0^{2,4}]octan-3-carboxamid;
(exo) (1R,2R,4S,5S)-N-(2-Amino-2-oxoethyl)tricyclo[3.2.1.0^{2,4}]octan-3-carboxamid;
2,4-Dimethylbicyclo[4.1.0]heptan-7-carboxamid;
N-(2-Amino-2-oxoethyl)-2,4-dimethylbicyclo[4.1.0]heptan-7-carboxamid;
2,4-Dimethylbicyclo[4.1.0]heptan-7-carboxamid;
(1S,2S,4S,6R,7S)-N-(2-Amino-2-oxoethyl)-2,4-dimethylbicyclo[4,1.0]heptan-7-carboxamid;
(2S,5R)-2-Isopropyl-5-methylbicyclo[4.1.0]heptan-7-carboxamid;
(2S,5R)-N-(2-Amino-2-oxoethyl)-2-isopropyl-5-methylbicyclo[4.1.0]heptan-7-carboxamid;
(endo) Bicyclo[6.1.0]nonan-9-carboxamid;
(endo) N-(2-Amino-2-oxoethyl)bicyclo[6.1.0]nonan-9-carboxamid;
(exo) Bicyclo[6.1.0]nonan-9-carboxamid;
(exo) N-(2-Amino-2-o-xoethyl)bicyclo[6.1.0]nonan-9-carboxamid;
2,7,7-Trimethyltricyclo[4.1.1.0^{2,4}]octan-3-carboxamid;
N-(2-Amino-2-oxoethyl)-2,7,7-trimethyltricyclo[4.1.1.0^{2,4}]octan-3-carboxamid;
1-Methylbicyclo[4.1.0]heptan-7-carboxamid;
N-(2-Amino-2-oxoethyl)-1-methylbicyclo[4.1.0]heptan-7-carboxamid;
(exo) Bicyclo[5.1.0]octan-8-carboxamid;
(exo) N-(2-Amino-2-oxoethyl)bicyclo[5.1.0]octan-8-carboxamid;
Bicyclo[3.1.0]hexan-6-carboxamid;
(exo) N-(2-Amino-2-oxoethyl)bicyclo[3.1.0]hexan-6-carboxamid;
4,7,7-Trimethyltricyclo[4.1.1.0^{2,4}]octan-3-carboxamid;
N-(2-Amino-2-oxoethyl)-4,7,7-trimethyltricyclo[4.1.1.0^{2,4}]octan-3-carboxamid;
3-tert-Butylbicyclo[4.1.0]heptan-7-carboxamid;
N-(2-Amino-2-oxoethyl)-3-tert-butylbicyclo[4,1.0]heptan-7-carboxamid;
1-Methylbicyclo[3,1.0]hexan-6-carboxamid;
N-(2-Amino-2-oxoethyl)-1-methylbicyclo[3.1.0]hexan-6-carboxamid;
1,5-Dimethylbicyclo[4.1.0]heptan-7-carboxamid oder
N-(2-Amino-2-oxoethyl)-1,5-dimethylbicyclo[4.1.0]heptan-7-carboxamid ist.

11. Verwendung gemäß Anspruch 1, worin
A Bicycloalkyl ist und
R₁ NR₃R₄ ist.

12. Verwendung gemäß Anspruch 1, worin
A Bicycloalkyl ist, worin das Bicycloalkyl wahlweise substituiert ist mit 1 oder 2 Alkylgruppen;
R₁ NR₃R₄ ist;
R₃ Wasserstoff ist;
R₄ Wasserstoff oder (NR₅R₆)Carbonylalkyl ist und
R₅ und R₆ Wasserstoff sind.

13. Verwendung gemäß Anspruch 12, worin die Verbindung mit der Formel (I)
(1S,3S,4S,7R)-3,8,8-Trimethyltricyclo[5.1.0.0^{3,5}]octan-4-carboxamid;
(1S,3S,4S,7R)-N-(2-Amino-2-oxoethyl)-3,8,8-trimethyltricyclo[5.1.0.0^{3,5}]octan-4-carboxamid;
(exo) (1aR,2R,2aS,5aR,6S,6aS)-Decahydro-2,6-methanocyclopropa[f]inden-1-carboxamid;
(exo) (1aR,2R,2aS,5aR,6S,6aS)-N-(2-Amino-2-oxoethyl)decahydro-2,6-methanocyclopropa[f]inden-1-carboxamid;
(1R,5S)-Tricyclo [3.3.0.0^{2,4}]oct-2(4)-en-3-carboxamid;
(1R,5S)-N-(2-Amino-2-oxoethyl)tricyclo[3.3.0.0^{2,4}]oct-2(4)-en-3-carboxamid;
Octahydro-1H-cyclopropa[a]pentalen-1-carboxamid;
N-(2-Amino-2-oxoethyl)octahydro-1H-cyclopropa[a]pentalen-1-carboxamid;
(1R,2R,4R,7R)-4,8,8-Trimethyltricyclo[5.1.0.0^{2,4}]octan-3-carboxamid oder
(1R,2R,4R,7R)-N-(2-Amino-2-oxoethyl)-4,8,8-trimethyltricyclo[5.1.0.0^{2,4}]octan-3-carboxamid ist.

14. Verwendung einer therapeutisch wirksamen Menge einer Verbindung mit der Formel (I), wie in Anspruch 1 definiert, für die Herstellung eines Medikaments zur Behandlung einer psychiatrischen Erkrankung bei einem Säugetier.

15. Verwendung einer therapeutisch wirksamen Menge einer Verbindung mit der Formel (I), wie in Anspruch 1 definiert, für die Herstellung eines Medikaments zur Behandlung von Schmerz bei einem Säugetier.

16. Verwendung einer therapeutisch wirksamen Menge einer Verbindung mit der Formel (I), wie in Anspruch 1 definiert, für die Herstellung eines Medikaments zur Behandlung einer Bewegungserkrankung bei einem Säugetier.

17. Verwendung einer therapeutisch wirksamen Menge einer Verbindung mit der Formel (I), wie in Anspruch 1 definiert, für die Herstellung eines Medikaments zur Bereitstellung von Nervenschutz bei einem Säugetier.

18. Verbindung mit der Formel (II) 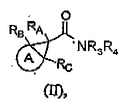 worin
A Cycloalkyl ist, worin das Cycloalkyl wahlweise substituiert ist mit 1 oder 2 Alkylgruppen;
R_{A}, R_{B} und R_{C} unabhängig Wasserstoff oder Alkyl sind;
R₃ Wasserstoff ist;
R₄ (NR₅R₆)Carbonylalkyl ist; und
R₅ und R₆ Wasserstoff sind.

19. Verbindung gemäß Anspruch 18, worin die Verbindung mit der Formel (II)
(exo) (1R,6S)-N-(2-Amino-2-oxoethyl)bicyclo[4.1.0]heptan-7-carboxamid;
N-(2-Amino-2-oxoethyl)-3-methylbicyclo[4.1.0]heptan-7-carboxamid;
(exo) (1R,2R,4S,5S)-N-(2-Amino-2-oxoethyl)tricyclo[3.2.1.0^{2,4}]octan-3-carboxamid;
N-(2-Amino-2-oxoethyl)-2,4-dimethylbicyclo[4.1.0]heptan-7-carboxamid;
(1S,2S,4S,6R,7S)-N-(2-Amino-2-oxoethyl)-2,4-dimethylbicylo[4.1.0]heptan-7-carboxamid;
(2S,5R)-N-(2-Amino-2-oxoethyl)-2-isopropyl-5-methylbicyclo[4.1.0]heptan-7-carboxamid;
(endo) N-(2-Amino-2-oxoethyl)bicyclo[6.1.0]nonan-9-carboxamid;
(exo) N-(2-Amino-2-oxoethyl)bicyclo[6.1.0]nonan-9-carboxamid;
N-(2-Amino-2-oxoethyl)-2,7,7-trimethyltricyclo[4.1.1.0^{2,4}]octan-3-carboxamid;
N-(2-Amino-2-oxoethyl)-1-methylbicyclo[4.1.0]heptan-7-carboxamid;
(exo) N-(2-Amino-2-oxoethyl)bicyclo[5.1.0]octan-8-carboxamid;
(exo) N-(2-Amino-2-oxoethyl)bicyclo[3.1.0]hexan-6-carboxamid;
N-(2-Amino-2-oxoethyl)-4,7,7-trimethyltricyclo[4.1.1.0^{2,4}]octan-3-carboxamid;
N-(2-Amino-2-oxoethyl)-3-tert-butylbicyclo[4.1.0]heptan-7-carboxamid;
N-(2-Amino-2-oxoethyl)-1-methylbicyclo[3.1.0]hexan-6-carboxamid oder
N-(2-Amino-2-oxoethyl)-1,5-dimethylbicyclo[4.1.0]heptan-7-carboxamid ist.

20. Verbindung mit der Formel (II) 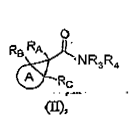 worin
A Bicycloalkyl ist, worin das Bicycloalkyl wahlweise substituiert ist mit 1 oder 2 Alkylgruppen; R_{A}, R_{B} und R_{C} unabhängig Wasserstoff oder Alkyl sind;
R₃ Wasserstoff ist;
R₄ (NR₅R₆)Carbonylalkyl ist; und
R₅ und R₆ Wasserstoff sind.

21. Verbindung gemäß Anspruch 20, worin die Verbindung mit der Formel (II)
(1S,3S,4S,7R)-N-(2-Amino-2-oxoethyl)-3,8,8-trimethyltricyclo[5.1.0.0^{3,5}]octan-4-carboxamid;
(exo) (1aR,2R,2aS,5aR,6S,6aS)-N-(2-Amino-2-oxoethyl)decahydro-2,6-methancyclopxopa[f]inden-1-carboxamid;
(1R,5S)-N-(2-Amino-2-oxoethyl)tricyclo[3.3.0.0^{2,4}]oct-2(4)-en-3-carboxamid;
N-(2-Amino-2-oxoethyl)octahydro-1H-cyclopropa[a]pentalen-1-carboxamid oder
(1R,2R,4R,7R)-N-(2-Amino-2-oxoethyl)-4,8,8-trimethyltricyclo[5.1.0.0^{2,4}]octan-3-carboxamid ist.

22. Verwendung einer therapeutisch wirksamen Menge einer Verbindung mit der Formel (I), wie in Anspruch 1 definiert, für die Herstellung eines Medikaments zur Behandlung von Neuropathie- und Entzündungsschmerz bei einem Säugetier.

## Revendications

1. Utilisation d'une quantité thérapeutiquement efficace d'un composé de formule (I) 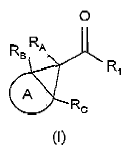 où
A est cycloalkyle ou bicycloalkyle ;
R_{A}, R_{B} et R_{C} sont indépendamment l'hydrogène ou alkyle ;
R₁ est OR₂ ou NR₃R₄ ;
R₂ est l'hydrogène ou alkyle ;
R₃ et R₄ sont indépendamment l'hydrogène, alcényle, alkyle, alcynyle, alcoxycarbonylalkyle, aryle, arylalkyle, carboxyalkyle, cycloalkyle, cycloalkylalkyle, hétérocycle, hétérocycloalkyle, hydroxyalkyle, (NR₅R₆)alkyle, (NR₅R₆)carbonylalkyle, ou 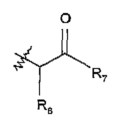 - ou
R₃ et R₄ combinés avec l'atome d'azote auquel ils sont liés forment un hétérocycle où l'hétérocycle est azépanyle, azétidinyle, aziridinyle, morpholinyle, pipérazinyle, pipéridinyle, pyrrolidinyle ou thiomorpholinyle ;
R₅ et R₆ sont indépendamment l'hydrogène, alcényle, alkyle, alcynyle, alcoxycarbonylalkyle, aryle, arylalkyle, cycloalkyle, cycloalkylalkyle, hétérocycle, hétérocyclealkyle ou hydroxyalkyle ;
R₇ est alcoxy, alkyle, hydroxy ou -NR₅R₆ ;
R₈ est alcényle, alcoxyalkyle, alcoxycarbonylalkyle, alkylthioalkyle, alcynyle, aryle, arylalkyle, carboxyalkyle, cycloalkyle, cycloalkylalkyle, hétérocycle, hétérocyclealkyle, hydroxyalkyle, mercaptoalkyle, (NR₅R₆)alkyle, (NR₅R₆)carbonylalkyle ou -CH₂)ₙNHC(=NH)NH₂ ; et
n est un entier de 1 à 6 ;
à condition que le composé de formule (I) soit différent de l'acide bicyclo[4.1.0]heptane-7-carboxylique,
pour la fabrication d'un médicament pour traiter la migraine, l'épilepsie ou un trouble bipolaire chez un mammifère.

2. Utilisation selon la revendication 1 où
A est cycloalkyle ; et
R₁ est OR₂.

3. Utilisation selon la revendication 1 où
A est cycloalkyle où le cycloalkyle est éventuellement substitué avec 1 ou 2 groupes alkyle ;
et
R₁ est OR₂.

4. Utilisation selon la revendication 3 où le composé de formule (I) est
l'acide 3-méthylbicyclo[4.1.0]heptane-7-carboxylique ;
l'acide (exo)(1R,2R,4S,5S)-tricyclo[3.2.1.0^{2,4}]octane-3-carboxylique
l'acide 2,4-diméthylbicyclo[4.1.0]heptane-7-carboxylique ;
l'acide (trans)2,4-diméthylbicyclo[4.1.0]heptane-7-carboxylique ;
l'acide (2S,5R)-2-isopropyl-5-méthylbicyclo[4.1.0]heptane-7-carboxylique ;
l'acide (endo)bicyclo[6.1.0]nonane-9-carboxylique ;
l'acide (exo)bicyclo[6.1.0]nonane-9-carboxylique ;
l'acide 2,7,7-triméthyitricyclo[4.1.1.0^{2,4}]octane-3-carboxylique ;
l'acide 1-méthylbicyclo[4.1.0]heptane-7-carboxylique ;
l'acide (exo)bicyclo[3.1.0]hexane-6-carboxylique ;
l'acide 4,7,7-triméthyltricyclo[4.1.1.0^{2,4}]octane-3-carboxylique ;
l'acide 3-tert-butylbicyclo[4.1.0]heptane-7-carboxylique ;
l'acide 1-méthylbicyclo[3.1.0]hexane-6-carboxylique ; ou
l'acide 1,5-diméthylbicyclo[4.1.0]heptane-7-carboxylique.

5. Utilisation selon la revendication 1 où
A est bicycloalkyle ; et
R₁ est OR₂.

6. Utilisation selon la revendication 1 où
A est bicycloalkyle où le bicycloalkyle est éventuellement substitué avec 1 ou 2 groupes alkyle ; et
R₁ est OR₂.

7. Utilisation selon la revendication 6 où le composé de formule (I) est
l'acide (1S,3S,5S,7R)-3,8,8-triméthyltricyclo[5.1.0.0^{3,5}]octane-4-carboxylique ;
l'acide (1S,3S,4R,7R)-3,8,8-triméthyltricyclo[5.1.0.0^{3,5}]octane-4-carboxylique ;
l'acide (exo)(1aR,2R,2aS,5aR,6S,6aS)-décahydro-2,6-méthanocyclopropa-[f]indène-1-carboxylique ;
l'acide (1R,5S)-tricyclo[3.3.0.0^{2,4}]oct-2(4)-ène-3-carboxylique ;
l'acide octahydro-1H-cyclopropa[a]pentalène-1-carboxylique ; ou
l'acide (1R,2R,4R,7R)-4,8,8-triméthyltricyclo[5.1.0.0^{2,4}]octane-3-carboxylique.

8. Utilisation selon la revendication 1 où
A est cycloalkyle ; et
R₁ est NR₃R₄.

9. Utilisation selon la revendication 1 où
A est cycloalkyle où le cycloalkyle est éventuellement substitué avec 1 ou 2 groupes alkyle ;
R₁ est NR₃R₄ ;
R₃ est l'hydrogène ;
R₄ est l'hydrogène ou (NR₅R₆)carbonylalkyle ; et
R₅ et R₆ sont l'hydrogène.

10. Utilisation selon la revendication 9 où le composé de formule (I) est
le (exo)(1R,6S)-bicyclo[4.1.0]heptane-7-carboxamide ;
le (exo)(1R,6S)-N-(2-amino-2-oxoéthyl)bicyclo[4.1.0]heptane-7-carboxamide ;
le 3-méthylbicyclo[4.1.0]heptane-7-carboxamide ;
le N-(2-amino-2-oxoéthyl)-3-méthylbicyclo[4.1.0]heptane-7-carboxamide ; le (exo)(1R,2R,4S,5S)-tricyclo[3.2.1.0^{2,4}]octane-3-carboxamide ;
le (exo)(1R,2R,4S,5S)-N-(2-amino-2-oxoéthyl)tricyclo[3.2.1.0^{2,4}]octane-3-carboxamide ;
le 2,4-diméthylbicyclo[4.1.0]heptane-7-carboxamide ;
le N-(2-amino-2-oxoéthyl)-2,4-diméthylbicyclo[4.1.0]heptane-7-carboxamide ;
le 2,4-diméthylbicyclo[4.1.0]heptane-7-carboxamide ;
le (1S,2S,4S,5R,7S)-N-(2-amino-2-oxoéthyl)-2,4-diméthylbicyclo[4.1.0]-heptane-7-carboxamide ;
le (2S,5R)-2-isopropyl-5-méthylbicyclo[4.1.0]heptane-7-carboxamide ;
le (2S,5R)-N-(2-amino-2-oxoéthyl)-2-isopropyl-5-méthylbicyclo[4.1.0]-heptane-7-carboxamide ;
le (endo)bicyclo[6.1.0]nonane-9-carboxamide ;
le (endo)N-(2-amino-2-oxoéthyl)bicyclo[6.1.0]nonane-9-carboxamide ;
le (exo)bicyclo[6.1.0]nonane-9-carboxamide ;
le (exo)N-(2-amino-2-oxoéthyl)bicyclo[6.1.0]nonane-9-carboxamide ;
le 2,7,7-triméthyltricyclo[4.1.1.0^{2,4}]octane-3-carboxamide ;
le N-(2-amino-2-oxoéthyl)-2,7,7-triméthyltricyclo[4.1.1.0^{2,4}]octane-3-carboxamide ;
le 1-méthylbicyclo[4.1.0]heptane-7-carboxamide ;
le N-(2-amino-2-oxoéthyl)-1-méthylbicyclo[4.1.0]heptane-7-carboxamide ;
le (exo)bicyclo[5.1.0]octane-8-carboxamide ;
le (exo)N-(2-amino-2-oxoéthyl)bicyclo[5.1.0]octane-8-carboxamide ;
le bicyclo[3.1.0]hexane-6-carboxamide ;
le (exo)N-(2-amino-2-oxoéthyl)bicyclo[3.1.0]hexane-6-carboxamide ;
le 4,7,7-triméthyltricyclo[4.1.1.0^{2,4}]octane-3-carboxamide ;
le N-(2-amino-2-oxoéthyl)-4,7,7-triméthyltricyclo[4.1.1.0^{2,4}]octane-3-carboxamide ;
le 3-tert-butylbicyclo[4.1.0]heptane-7-carboxamide ;
le N-(2-amino-2-oxoéthyl)-3-tert-butylbicyclo[4.1.0]heptane-7-carboxamide ;
le 1-méthylbicyclo[3.1.0]hexane-6-carboxamide ;
le N-(2-amino-2-oxoéthyl)-1-méthylbicyclo[3.1.0]heptane-6-carboxamide ;
le 1,5-diméthylbicyclo[4.1.0]heptane-7-carboxamide ; ou le N-(2-amino-2-oxoéthyl)-1,5-diméthylbicyclo[4.1.0]heptane-7-carboxamide.

11. Utilisation selon la revendication 1 où
A est bicycloalkyle ; et
R₁ est NR₃R₄.

12. Utilisation selon la revendication 1 où
A est bicycloalkyle où le bicycloalkyle est éventuellement substitué avec 1 ou 2 groupes alkyle ;
R₁ est NR₃R₄ ;
R₃ est l'hydrogène ;
R₄ est l'hydrogène ou (NR₅R₆)carbonylalkyle ; et
R₅ et R₆ sont l'hydrogène.

13. Utilisation selon la revendication 12 où le composé de formule (I) est
le (1S,3S,4S,7R)-3,8,8-triméthyltricyclo[5.1.0.0^{3,5}]octane-4-carboxamide ;
le (15,35,4S,7R)-N-(2-amino-2-oxoéthyl)-3,8,8-triméthyltricyclo-[5.1.0.0^{3,5}]octane-4-carboxamide ;
le (exo)(1aR,2R,2aS,5aR,6S,6aS)-décahydro-2,6-méthanocyclopropa[f]-indène-1-carboxamide ;
le (exo)(1aR,2R,2aS,5aR,6S,6aS)-N-(2-amino-2-oxoéthyl)décahydro-2,6-méthanocyclopropa[f]indène-1-carboxamide ;
le (1R,5S)-tricyclo[3.3.0.0^{2,4}]oct-2(4)-ène-3-carboxamide ;
le (1R,5S)-N-(2-amino-2-oxoéthyl)tricyclo[3.3.0.0^{2,4}]oct-2(4)-ène-3-carboxamide ;
l'octahydro-1H-cyclopropa[a]pentalène-l-carboxamide ;
le N-(2-amino-2-oxoéthyl)octahydro-1H-cyclopropa[a]pentalène-1-carboxamide ;
le (1R,2R,4R,7R)-4,8,8-triméthyltricyclo[5.1.0.0^{2,4}]octane-3-carboxamide ;
ou
le (1R,2R,4R,7R)-N-(2-amino-2-oxoéthyl)-4,8,8-triméthyltricyclo-[5.1.0.0^{2,4}]octane-3-carboxamide.

14. Utilisation d'une quantité thérapeutiquement efficace d'un composé de formule (I), tel que défini dans la revendication 1, pour la fabrication d'un médicament pour traiter un trouble psychiatrique chez un mammifère.

15. Utilisation d'une quantité thérapeutiquement efficace d'un composé de formule (I), tel que défini dans la revendication 1, pour la fabrication d'un médicament pour traiter la douleur chez un mammifère.

16. Utilisation d'une quantité thérapeutiquement efficace d'un composé de formule (I), tel que défini dans la revendication 1, pour la fabrication d'un médicament pour traiter un trouble des mouvements chez un mammifère.

17. Utilisation d'une quantité thérapeutiquement efficace d'un composé de formule (I), tel que défini dans la revendication 1, pour la fabrication d'un médicament pour conférer une neuroprotection à un mammifère.

18. Composé de formule (II) 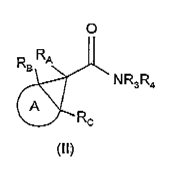 où
A est cycloalkyle où le cycloalkyle est éventuellement substitué avec 1 ou 2 groupes alkyle ;
R_{A}, R_{B} et R_{C} sont indépendamment l'hydrogène ou alkyle ;
R₃ est l'hydrogène ;
R₄ est (NR₅R₆)carbonylalkyle ; et
R₅ et R₆ sont l'hydrogène.

19. Composé selon la revendication 18 où le composé de formule (II) est
le (exo)(1R,6S)-N-(2-amino-2-oxoéthyl)bicyclo[4.1.0]heptane-7-carboxamide ;
le N-(2-amino-2-oxoéthyl)-3-méthylbicyclo[4.1.0]heptane-7-carboxamide ;
le (exo)(1R,2R,4S,5S)-N-(2-amino-2-oxoéthyl)tricyclo[3.2.1.0^{2,4}]octane-3-carboxamide ;
le N-(2-amino-2-oxoéthyl)-2,4-diméthylbicyclo[4.1.0]heptane-7-carboxamide ;
le (1S,2S,4S,6R,7S)-N-(2-amino-2-oxoéthyl)-2,4-diméthylbicyclo[4.1.0]-heptane-7-carboxamide ;
le (2S,5R)-N-(2-amino-2-oxoéthyl)-2-isopropyl-5-méthylbicyclo[4.1.0]-heptane-7-carboxamide ;
le (endo)N-(2-amino-2-oxoéthyl)bicyclo[6.1.0]nonane-9-carboxamide ;
le (exo)N-(2-amino-2-oxoéthyl)bicyclo[6.1.0]nonane-9-carboxamide ;
le N-(2-amino-2-oxoéthyl)-2,7,7-triméthyltricyclo[4.1.1.0^{2,4}]octane-3-carboxamide ;
le N-(2-amino-2-oxoéthyl)-1-méthylbicyclo[4.1.0]heptane-7-carboxamide ;
le (exo)N-(2-amino-2-oxoéthyl)bicyclo[5.1.0]octane-8-carboxamide ;
le (exo)N-(2-amino-2-oxoéthyl)bicyclo[3.1.0]hexane-6-carboxamide ;
le N-(2-amino-2-oxoéthyl)-4,7,7-triméthyltricyclo[4.1.1.0^{2,4}]octane-3-carboxamide ;
le N-(2-amino-2-oxoéthyl)-3-tert-butylbicyclo[4.1.0]heptane-7-carboxamide ;
le N-(2-amino-2-oxoéthyl)-1-méthylbicyclo[3.1.0]hexane-6-carboxamide ; ou
le N-(2-amino-2-oxoéthyl)-1,5-diméthylbicyclo[4.1.0]heptane-7-carboxamide.

20. Composé de formule (II) 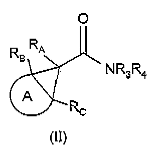 où
A est bicycloalkyle où le bicycloalkyle est éventuellement substitué avec 1 ou 2 groupes alkyle ;
R_{A}, R_{B} et R_{C} sont indépendamment l'hydrogène ou alkyle ;
R₃ est l'hydrogène ;
R₄ est (NR₅R₆)carbonylalkyle ; et
R₅ et R₆ sont l'hydrogène.

21. Composé selon la revendication 20 où le composé de formule (II) est
le (1S,3S,4S,7R)-N-(2-amino-2-oxoéthyl)-3,8,8-triméthyltricyclo-[5.1.0.0^{3,5}]octane-4-carboxamide ;
le (exo)(1aR,2R,2aS,5aR,6S,6aS)-N-(2-amino-2-oxoéthyl)décahydro-2,6-méthanocyclopropa[f]indène-1-carboxamide ;
le (1R,5S)-N-(2-amino-2-oxoéthyl)tricyclo[3.3.0.0^{2,4}]oct-2(4)ène-3-carboxamide ;
le N-(2-amino-2-oxoéthyl)octahydro-1H-cyclopropa[a]pentalène-1-carboxamide ; ou
le (1R,2R,4R,7R)-N-(2-amino-2-oxoéthyl)-4,8,8-triméthyltricyclo-[5.1.0.0^{2,4}]octane-3-carboxamide.

22. Utilisation d'une quantité thérapeutiquement efficace d'un composé de formule (I), tel que défini dans la revendication 1, pour la fabrication d'un médicament pour traiter la douleur neuropathique et inflammatoire chez un mammifère.
